# EUROPEAN PATENT APPLICATION

(11) **EP 2 351 722 A1**
(43) Date of publication of application: **03.08.2011**
(21) Application number: 09824722.4
(22) Date of filing: 27.10.2009
(51) Int. Cl.: C07C 2/34, B01J 31/14, B01J 31/22, C07C 7/04, C07C 7/163, C07C 9/22, C07C 11/02, C10M 107/02, C10M 107/10, C07B 61/00, C10N 20/04

(54) **METHOD FOR PRODUCING -OLEFIN OLIGOMER, -OLEFIN OLIGOMER, AND LUBRICATING OIL COMPOSITION**

(30) Priority: 04.11.2008 JP 2008283340; 26.12.2008 JP 2008334860
(71) Applicant: Idemitsu Kosan Co., Ltd., Chiyoda-ku Tokyo 100-8321 (JP)
(72) Inventor: FUJIKAWA, Shinjiro, Ichihara-shi Chiba 299-0193 (JP); YOKOTA, Kiyohiko, Ichihara-shi Chiba 299-0193 (JP); OKANO, Masaki, Ichihara-shi Chiba 299-0193 (JP); TSUJI, Minako, Ichihara-shi Chiba 299-0193 (JP)
(74) Representative: Vossius & Partner
(86) International application number: PCT/JP2009/068406
(87) International publication number: WO 2010/053022

(57) **Abstract**

A method of producing an α-olefin oligomer composition of the present invention is a method of producing an α-olefin oligomer, composition, the method including the step of causing the molecules of an α-olefin to react with each other with a specific catalyst in the presence of hydrogen.

## Description

### Technical Field

The present invention relates to a method of producing an α-olefin oligomer, an α-olefin oligomer obtained by the production method, and a lubricating oil composition containing the α-olefin oligomer.

### Background Art

α-olefin oligomers have been heretofore used in, for example, base oils for lubricating oils because the oligomers have, for example, the following characteristics: each of the oligomers shows good fluidity at low temperatures, has relatively high thermal stability and relatively high oxidation stability, is lost in a small amount by its evaporation at high temperatures, and has a relatively high viscosity index. The α-olefin oligomers have been conventionally produced with acid catalyst such as BF₃ and AlCl₃, and an α-olefin oligomer having a target degree of polymerization is obtained by distillation after the reaction. For example, with regard to the oligomers of 1-decane, the trimer, tetramer, and pentamer of 1-decene have been finding use in lubricating oil applications. A method of producing each of those oligomers has involved the following problem: an isomerization reaction is apt to occur at the time of an oligomerization reaction, so the molecules of the resultant oligomer do not have a uniform structure, and the oligomer is a mixture of various structures.

With regard to a method of producing an α-olefin oligomer, a method of producing an α-olefin oligomer involving the use of a metallocene catalyst has been recently known, and the method has improved the uniformity of the structures of the molecules of the oligomer.

For example, Patent Document 1 discloses a method of producing an α-olefin oligomer with a catalyst system using a metallocene compound and a methylaluminoxane as an example of the production of an α-olefin oligomer involving the use of a metallocene catalyst. However, the method involves, for example, the following problems: a ratio of aluminum to an α-olefin is high, so the production efficiency of the oligomer is low; the decalcification efficiency of the catalyst is poor, so a large amount of an acid or alkali is needed, and the need adversely affects an environment to a large extent; the quality of the product deteriorates owing to an element remaining in the product; and a large amount of toluene is used in a reaction for the production of the oligomer, so the quality of the product deteriorates owing to the inclusion of toluene in the product. Patent Documents 2 and 3 disclose production examples in each of which an improvement in activity of a catalyst formed of a transition metal compound and a methylaluminoxane is attained by reducing the amount of the transition metal compound and using a large amount of the methylaluminoxane. However, the methods each involve a problem in a decalcifying step because a large amount of aluminum is used. Patent Documents 4 and 5 each disclose a method of producing an α-olefin oligomer with a catalyst system using a metallocene compound and a methylaluminoxane, and the usage of each of the metallocene compound and the methylaluminoxane has been reduced in the method. However, the methods each involve the following problems: the catalyst system shows low catalytic activity, and an oligomer yield is poor. In addition, neither Patent Document 4 nor Patent Document 5 discloses excellent catalytic activity which a specific transition metal compound shows in the presence of hydrogen. Patent Document 6 discloses a method involving adding H₂ as a method of improving catalytic activity, but the effect of the method is insufficient. In addition, most of the oligomers obtained by the method are each a dimer, and the yield in which oligomers each of which is a trimer or more preferable for use in a lubricating oil are obtained is low.

### Prior Art Document

### Patent Document

[Patent Document 1] JP 2005-501957 A
[Patent Document 2] JP 05-39229 A
[Patent Document 3] JP 07-133234 A
[Patent Document 4] US 2001/041817 A
[Patent Document 5] US 2001/041818 A
[Patent Document 6] JP 2006-225348 A

### Summary of the Invention

### Problems to be solved by the Invention

The present invention has been made in view of the above circumstances, and an object of the present invention is to provide a method of producing an α-olefin oligomer in which a catalyst shows excellent catalytic activity.
Another object of the present invention is to provide a method of producing an α-olefin oligomer composition by which the amount of a metal used in a catalyst can be reduced as compared to a conventional one and by which a trimer, a tetramer, and a pentamer suitable for use in lubricating oils can each be produced with a high selectivity.

### Means for solving the Problems

The inventors of the present invention have made extensive studies. As a result, the inventors have found that the above problems can be solved by using a catalyst formed by using a specific transition metal compound. The present invention has been completed on the basis of such finding.
That is, the present invention provides the following.
(1) A method of producing an α-olefin oligomer composition, the method including the step of causing molecules of an α-olefin to react with each other in the presence of hydrogen with a catalyst formed by using the following components (A) and (B):
   (A) a transition metal compound represented by a general formula (I):

      {C₅R¹R²R³R⁴(A¹R^{a}R^{b}R^{c})} {C₅R⁵R⁶R⁷R⁸(A²R^{d}R^{e}R^{f}) }MXY (I)

      where R¹ to R⁸, (A¹R^{a}R^{b}R^{c}), and (A²R^{d}R^{e}R^{f}) each represent a substituent bonded to a cyclopentadienyl group, R¹ to R⁸ each independently represent a hydrogen atom or a hydrocarbon group having 1 to 10 carbon atoms, R^{a} to R^{f} each independently represent a hydrocarbon group having 1 to 10 carbon atoms, and two or more groups selected from R^{a}, R^{b}, and R^{c} may be bonded to each other to form a ring, or two or more groups selected from R^{d}, R^{e}, and R^{f} may be bonded to each other to form a ring, A¹ and A² each independently represent an element belonging to Group 14 of the periodic table, M represents a transition element belonging to Group 4 of the periodic table, and X and Y each represent a covalent ligand or an ionic bond ligand; and
   (B) a co-catalyst containing aluminum,
(2) A method of producing an α-olefin oligomer composition, the method including the step of causing molecules of an α-olefin to react with each other with a catalyst formed by using the following components (A) and (B) in an aluminum amount in a range of 1.89×10⁻⁵ to 5.67×10⁻² mol per mol of the α-olefin at a hydrogen pressure in a range of 1 to 50 kPa (G):
   (A) a transition metal compound represented by a general formula (I) :

      {C₅R¹R²R³R⁴(A¹R^{a}R^{b}R^{c})} {C₅R⁵R⁶R⁷R⁸(A²R^{d}R^{e}R^{f})}MXY (I)

      where R¹ to R⁸, (A¹R^{a}R^{b}R^{c}), and (A²R^{d}R^{e}R^{f}) each represent a substituent bonded to a cyclopentadienyl group, R¹ to R⁸ each independently represent a hydrogen atom or a hydrocarbon group having 1 to 10 carbon atoms, R^{a} to R^{f} each independently represent a hydrocarbon group having 1 to 10 carbon atoms, and two or more groups selected from R^{a}, R^{b}, and R^{c} may be bonded to each other to form a ring, or two or more groups selected from R^{d}, R^{e}, and R^{f} may be bonded to each other to form a ring, A¹ and A² each independently represent an element belonging to Group 14 of the periodic table, M represents a transition element belonging to Group 4 of the periodic table, and X and Y each represent a covalent ligand or an ionic bond ligand; and
   (B) a co-catalyst containing aluminum,
(3) The method of producing an α-olefin oligomer composition according to the above item (2) , in which the co-catalyst containing aluminum as the component (B) includes an organic aluminum oxy compound and/or an organic aluminum compound,
(4) The method of producing an α-olefin oligomer composition according to the above item (2) , in which the transition metal compound as the component (A) includes a transition metal compound represented by a general formula (II) :

   {C₅H₄(A³R^{g}R^{h}Rⁱ) }₂ZrCl₂ (II)

   where R^{g}, R^{h}, and Rⁱ each independently represent a hydrocarbon group having 1 to 10 carbon atoms, and two or more of the groups may be bonded to each other to form a ring, and A³ represents an element belonging to Group 14 of the periodic table,
(5) The method of producing an α-olefin oligomer composition according to the above item (2), in which the transition metal compound as the component (A) includes a transition metal compound represented by a general formula (III):

   {C⁵H₄ (CR^{j}R^{k}R^{l})}ZrCl₂ (III)

   where R^{j}, R^{k}, and R^{l} each independently represent a hydrocarbon group having 1 to 10 carbon atoms, and two or more of the groups may be bonded to each other to form a ring,
(6) The method of producing an α-olefin oligomer composition according to the above item (2) , in which the transition metal compound as the component (A) includes a transition metal compound represented by a formula (IV);

   {C⁵H₄(CMe₃)}₂ZrCl₂ (IV),
(7) The method of producing an α-olefin oligomer composition according to the above item (2), in which the α-olefin includes 1-decene,
(8) The method of producing an α-olefin oligomer composition according to the above item (2), in which the α-olefin oligomer composition includes an α-olefin oligomer composition satisfying the following relationships: A>B and A>C when a ratio of a trimer to a dimer on a mass basis is represented by A, a ratio of a tetramer to the trimer on a mass basis is represented by B, and a ratio of a pentamer to the tetramer on a mass basis is represented by C,
(9) An α-olefin oligomer composition obtained by the method according to the above item (2),
(10) A method of producing a hydrogenated α-olefin oligomer composition, the method including the step of subjecting an α-olefin oligomer composition obtained by the method according to the above item (2) to a hydrogenation treatment,
(11) A hydrogenated α-olefin oligomer composition obtained by the method according to the above item (10),
(12) A lubricating oil composition including the α-olefin oligomer composition according to the above item (9) and/or the hydrogenated α-olefin oligomer composition according to the above item (11),
(13) A method of producing an α-olefin oligomer, the method including the step of polymerizing molecules of an α-olefin having 3 to 14 carbon atoms in the presence of hydrogen by using a metallocene compound represented by a general formula (VII) and a methylaluminoxane at a molar ratio (methylaluminoxane/metallocene compound) of 15 to 110:

   (RC₅H₄)₂MX₂ (VII)

   where R represents a hydrogen atom or a hydrocarbon group having 1 to 10 carbon atoms, M represents a transition metal element belonging to Group 4 of the periodic table, and X represents a covalent ligand or an ionic bond ligand,
(14) The production method according to the above item (13), in which a ratio at which the metallocene compound represented by the general formula (VII) and the α-olefin are blended with each other [metallocene compound (mmol)/α-olefin (L)] falls within a range of 0.01 to 0.4,
(15) The production method according to the above item (13), in which M in the general formula (VII) represents zirconium,
(16) The production method according to the above item (13), in which a hydrogen pressure falls within a range of 0.1 to 50 kPa,
(17) The production method according to the above item (13), in which the α-olefin having 3 to 14 carbon atoms includes an α-olefin selected from 1-octene, 1-decene, and 1-dodecene,
(18) The production method according to the above item (13), in which the α-olefin having 3 to 14 carbon atoms includes 1-decene,
(19) The production method according to the above item (13), in which a selectivity of a trimer or more is 50% or more,
(20) An α-olefin oligomer obtained by the production method according to the above item (13),
(21) A method of producing a purified hydrogenated α-olefin oligomer, the method including the steps of: producing an α-olefin oligomer by the production method according to the above item (13); hydrogenating the α-olefin oligomer to produce a hydrogenated α-olefin oligomer; and distilling the hydrogenated α-olefin oligomer to obtain a fraction having a kinematic viscosity at 100°C of 3 to 35 mm²/s,
(22) A purified hydrogenated α-olefin oligomer obtained by the production method according to the above item (21).

### Effect of the Invention

According to the present invention, there is provided a method of producing an α-olefin oligomer in which a catalyst shows excellent catalytic activity.
In addition, there is provided a method of producing an α-olefin oligomer composition by which the amount of a catalyst can be reduced and a trimer, a tetramer, and a pentamer can each be produced with a high selectivity. When the method is employed, a production cost for the composition can be suppressed by virtue of the reduction of the amount of the catalyst, and a reduction in quality of the product caused by a catalyst residue is avoided. Further, a trimer, a tetramer, and a pentamer can each be produced with a high selectivity, so the yield in which components useful in lubricating oils are obtained is high. In addition, the production of a component which is a hexamer or more is suppressed, so an excessive increase in viscosity of the composition is prevented, and an energy loss in a hydrogenating step or distilling step can be reduced.

### Best Mode for carrying out the Invention

A method of producing an α-olefin oligomer of the present invention is a method of producing an α-olefin oligomers, the method including the step of causing the molecules of an α-olefin to react with each other with a specific catalyst in the presence of hydrogen.

In the catalyst used in the present invention, a transition metal compound represented by the general formula (I) is used as the component (A):

{C₅R¹R²R³R⁴ (A¹R^{a}R^{b}R^{c})} {C₅R⁵R⁶R⁷R⁸ (A²R^{d}R^{e}R^{f}) }MXY (I)

In the general formula (I): R¹ to R⁸, (A¹R^{a}R^{b}R^{c}), and (A²R^{d}R^{e}R^{f}) each represent a substituent bonded to a cyclopentadienyl group; R¹ to R⁸ each independently represent a hydrogen atom or a hydrocarbon group having 1 to 10 carbon atoms; R^{a} to R^{f} each independently represent a hydrocarbon group having 1 to 10 carbon atoms, and two or more groups selected from R^{a}, R^{b}, and R^{c} may be bonded to each other to form a ring, or two or more groups selected from R^{d}, R^{e}, and R^{f} may be bonded to each other to form a ring; A¹ and A² each independently represent an element belonging to Group 14 of the periodic table such as carbon, silicon, or germanium; M represents a transition element belonging to Group 4 of the periodic table such as titanium, zirconium, or hafnium, and zirconium is preferable out of the elements; and X and Y each represent a covalent ligand or an ionic bond ligand such as a hydrogen atom, a halogen atom, a hydrocarbon group having 1 to 20 (preferably 1 to 10) carbon atoms, an alkoxy group having 1 to 20 (preferably 1 to 10) carbon atoms, an amino group, a phosphorus-containing hydrocarbon group having 1 to 20 (preferably 1 to 12) carbon atoms (such as a diphenylphosphine group), a silicon-containing hydrocarbon group having 1 to 20 (preferably 1 to 12) carbon atoms (such as a trimethylsilyl group), or a boron compound containing a hydrocarbon group having 1 to 20 (preferably 1 to 12) carbon atoms or a halogen (such as B(C₆H₅)₄ or BF₄), and a hydrogen atom, a halogen atom, a hydrocarbon group, and an alkoxy group are preferable out of these ligands, and X and Y may be identical to or different from each other.

As described above, the catalyst used in the present invention contains such bulky substituents as represented by (A¹R^{a}R^{b}R^{c}) and (A²R^{d}R^{e}R^{f}). When the bulky substituents are present, the polymerization of the molecules of the α-olefin can result in the production of each of a trimer, a tetramer, and a pentamer suitable for use in lubricating oils with a high selectivity. A substituent represented by (A¹R^{a}R^{b}R^{ç}) or (A²R^{d}R^{e}R^{f}) is preferably CR₃, in which C is a carbon atom and R is a hydrocarbon group having 1 to 10 carbon atoms, or particularly preferably CMe₃.

As the transition metal compound, exemplified is a transition metal compound represented by the general formula (II) :

{C₅H₄ (A³R^{g}R^{h}Rⁱ) }₂ZrCl₂ (II)

where R^{g}, R^{h}, and Rⁱ each independently represent a hydrocarbon group having 1 to 10 carbon atoms, and 2 groups or more may be bonded to each other to form a ring, and A³ represents an element belonging to Group 14 of the periodic table,
specifically exemplified is a transition metal compound represented by the formula (III):

{C₅H₄ (CR^{j}R^{k}R^{l}) }₂ZrCl₂ (III)

where R^{j}, R^{k}, and R^{l} each independently represent a hydrocarbon group having 1 to 10 carbon atoms, and 2 groups or more may be bonded to each other to form a ring,
and more specifically exemplified is a transition metal compound represented by the general formula (IV):

{C₅H₄(CMe₃)}₂ZrCl₂ (IV).

In the general formula (IV) Me represents a methyl group.

Specific examples of the transition metal compound represented by the general formula (I) are described below.
Examples of the bis(monosubstituted cyclopentadienyl)-zirconocene include bis(t-butylcyclopentadienyl)zirconium dichloride, bis(t-pentylcyclopentadienyl)zirconium dichloride, bis((2-methylpentane-2-yl)cyclopentadienyl)zirconium dichloride, bis((2,4-dimethylpentane-2-yl)cyclopentadienyl)zirconium dichloride, bis((2,3-dimethylbutane-2-yl)cyclopentadienyl)zirconium dichloride, bis((2,3,3-trimethylbutane-2-yl)cyclopentadienyl)zirconium dichloride, bis((3-methylpentane-3-yl)cyclopentadienyl)zirconium dichloride, bis((3-methylhexane-3-yl)cyclopentadienyl)zirconium dichloride, bis((3,5-dimethylhexane-3-yl)cyclopentadienyl)zirconium dichloride, bis((2,2,3-trimethylpentane-3-yl)cyclopentadienyl)zirconium dichloride, bis((2,3-dimethylpentane-3-yl)cyclopentadienyl)zirconium dichloride, bis((3-ethylpentane-3-yl)cyclopentadienyl)zirconium dichloride, bis((3-ethylhexane-3-yl)cyclopentadienyl)zirconium dichloride, bis((3-ethyl-5-methylhexane-3-yl)cyclopentadienyl)zirconium dichloride, bis((3-ethyl-2-methylpentane-3-yl)cyclopentadienyl)zirconium dichloride, bis((3-ethyl-2,2-dimethylpentane-3-yl)cyclopentadienyl)zirconi um dichloride, bis((1-methylcyclohexyl)cyclopentadienyl)zirconium dichloride, bis((1-ethylcyclohexyl)cyclopentadienyl)zirconium dichloride, bis((1-isopropylcyclohexyl)cyclopentadienyl)zirconium dichloride, bis((1-t-butylcyclohexyl)cyclopentadienyl)zirconium dichloride, bis((1-(2,3-dimethylbutane-2-yl)cyclohexyl)cyclopentadienyl)zi rconium dichloride, bis((1-methylcyclopentyl)cyclopentadienyl)zirconium dichloride, bis((1-ethyleyclopentyl)cyclopentadienyl)zircornium dichloride, bis((1-isopropylcyclopentyl)cyclopentadienyl)zirconium dichloride, bis((1-t-butylcyclopentyl)cyclopentadienyl)zirconium dichloride, bis((1-(2,3-dimethylbutane-2-yl)cyclopentyl)cyclopentadienyl)z irconium dichloride, bis((2-phenylpropane-2-yl)cyclopentadienyl)zirconium dichloride, bis((2-phenylbutane-2-yl)cyclopentadienyl)zirconium dichloride, bis(3-phenylpentane-3-yl)cyclopentadienyl)zirconium dichloride, bis((2-(2-indenyl)propane-2-yl)cyclopentadienyl)zirconium dichloride, bis((2-(2-indenyl)butane-2-yl)cyclopentadienyl)zirconium dichloride, bis((3-(2-indenyl)pentane-3-yl)cyclopentadienyl)zirconium dichloride, bis((2-(2-indenyl)propane-2-yl)cyclopentadienyl)zirconium dichloride, bis((2-indenylbutane-2-yl)cyclopentadienyl)zirconium dichloride, bis((3-indenylpentane-3-yl)cyclopentadienyl)zirconium dichloride, bis(1,1-diphenylethyl)cyclopentadienyl)zirconium dichloride, bis(1,1-diphenylpropyl)cyclopentadienyl)zirconium dichloride, and bis((1,1-diphenylbutyl)cyclopentadienyl)zirconium dichloride.

Examples of the bis(disubstituted cyclopentadienyl)-zirconocene include bis(1-methyl-3-t-butylcyclopentadienyl)zirconium dichloride, bis(1-methyl-3-t-pentylcyclopentadienyl)zirconium dichloride, bis(1-methyl-3-(2-methylpentane-2-yl)cyclopentadienyl)zirconiu m dichloride, bis(1-methyl-3-(2,4-dimethylpentane-2-yl)cyclopentadienyl)zirc onium dichloride, bis(1-methyl-3-(2,3-dimethylbutane-2-yl)cyclopentadienyl)zirco nium dichloride, bis(1-methyl-3-(2,3,3-trimethylbutane-2-yl)cyclopentadienyl)zi rconium dichloride, bis(1-methyl-3-(3-methylpentane-3-yl)cyclopentadienyl)zirconiu m dichloride, bis(1-methyl-3-(3-methylhexane-3-yl)cyclopentadienyl)zirconium dichloride, bis(1-methyl-3-(3,5-dimethylhexane-3-yl)cyclopentadienyl)zirco nium dichloride, bis(1-methyl-3-(3,4-dimethylpentane-3-yl)cyclopentadienyl)zirc onium dichloride, bis(1-methyl-3-(3,4,4-trimethylpentane-3-yl)cyclopentadienyl)z irconium dichloride, bis((1-methyl-3-ethylpentane-3-yl)cyclopentadienyl)zirconium dichloride, bis((1-methyl-3-ethylhexane-3-yl)cyclopentadienyl)zirconium dichloride, bis((1-methyl-(3-ethyl-5-methyl)hexane-3-yl)cyclopentadienyl)z irconium dichloride, bis((1-methyl-(3-ethyl-4-methyl)pentane-3-yl)cyclopentadienyl) zirconium dichloride, bis((1-methyl-(3-ethyl-4,4-dimethyl)pentane-3-yl)cyclopentadie nyl)zirconium dichloride, bis ((1-methyl-3-(1-methylcyclohexyl)cyclopentadienyl)zirconium dichloride, bis((1-methyl-3-(1-ethylcyclohexyl)cyclopentadienyl)zirconium dichloride, bis((1-methyl-3-(1-isopropylcyclohexyl)cyclopentadienyl)zircon ium dichloride, bis((1-methyl-3-(1-t-butylcyclohexyl)cyclopentadienyl)zirconiu m dichloride, bis((1-methyl-3-(2,3-dimethylbutane-2-yl)cyclohexyl)cyclopenta dienyl)zirconium dichloride, bis((1-methyl-3-(1-methylcyclopentyl)cyclopentadienyl)zirconiu m dichloride, bis((1-methyl-3-(1-ethylcyclopentyl)cyclopentadienyl)zirconium dichloride, bis((1-methyl-3-(1-isopropylcyclopentyl)cyclopentadienyl)zirco nium dichloride, bis((1-methyl-3-(1-t-butylcyclopentyl)cyclopentadienyl)zirconi um dichloride, and bis((1-methyl-3-(2,3-dimethylbutane-2-yl)cyclapentyl)cyclopent adienyl)zirconium dichloride.

Examples of the bis(trisubstituted cyclopentadienyl)-zirconocene include bis(1,2-dimethyl-3-t-butylcyclopentadienyl) zirconium dichloride, bis(1,2-dimethyl-3-t-pentylcyclopentadienyl)zirconium dichloride, bis(1,2-dimethyl-3-(2-methylpentane-2-yl)cyclopentadienyl)zirc onium dichloride, bis(1,2-dimethyl-3-(2,4-dimethylpentane-2-yl)cyclopentadienyl) zirconium dichloride, bis(1,2-dimethyl-3-(2,3-dimethylbutane-2-yl)cyclapentadienyl)z irconium dichloride, bis(1,2-dimethyl-3-(2,3,4-trimethylbutane-2-yl)cyclopentadieny l)zirconium dichloride, bis(1,2-dimethyl-3-(3-methylpentane-3-yl)cyclopentadienyl)zirc onium dichloride, bis(1,2-dimethyl-3-(3-methylhexane-3-yl)cyclopentadienyl)zirco nium dichloride, bis(1,2-dimethyl-3-(2-methylpentane-2-yl)cyclopentadienyl)zirc onium dichloride, bis(1,2-dimethyl-3-(2,4-dimethylpantane-2-yl)cyclopentadienyl) zirconium dichloride, bis(1,2-dimethyl-3-(2,3-dimethylbutane-2-yl)cyclopentadienyl)z irconium dichloride, bis(1,2-dimethyl-3-(2,3,4-trimethylbutane-2-yl)cyclopentadieny l)zirconium dichloride, bis(1,2-dimethyl-3-(3-ethylpentane-3-yl)cyclopentadienyl)zirco nium dichloride, bis(1,2-dimethyl-3-(3-ethylhexane-3-yl)cyclopentadienyl)zircon ium dichloride, bis(1,2-dimethyl-3-(3-ethyl-5-methylhexane-3-yl)cyclopentadien yl)zirconium dichloride, bis((1,2-dimethyl-3-(3-ethyl-4-methylpentane-3-yl)cyclopentadie nyl)zirconium dichloride, bis(1,2-dimethyl-3-(3-ethyl-4,4-dimethylpentane-3-yl)cyclopent adienyl)zirconium dichloride, bis(1,3-dimethyl-3-(1-methylcyclohexyl)cyclopentadienyl)zircon ium dichloride, bis(1,3-dimethyl-3-(1-ethylcyclohexyl)cyclopentadienyl)zirconi um dichloride, bis(1,3-dimethyl-3-(1-isopropylcyclohexyl)cyclopentadienyl)zir conium dichloride, bis(1,3-dimethyl-3-(1-t-butylcyclohexyl)cyclopentadienyl)zirco nium dichloride, bis(1,3-dimethyl-3-(2,3-dimethylbutane-2-yl)cyclohexyl)cyclope ntadienyl)zirconium dichloride, bis(1,3-dimethyl-3-(1-methylcyclopentyl)cyclopentadienyl)zirco nium dichloride, bis((1,3-dimethyl-3-(1-ethylcyclopentyl)cyclopentadienyl)zirco 1 nium dichloride, bis(1,3-dimethyl-3-(1-isopropylcyclopentyl)cyclopentadienyl)zi rconium dichloride, bis(1,3-dimethyl-3-(1-t-butylcyclopentyl)cyclopentadienyl)zirc onium dichloride, and bis((1,3-dimethyl-3-(2,3-dimethylbutane-2-yl) cyclopentyl)cyclopentadienyl)zirconium dichloride.

Further, exemplified are compounds which a chlorine atom of the above-mentioned compounds is substituted by a bromine atom, an iodine atom, a hydrogen atom, a methyl group, a phenyl group, a methoxy group, or the like. In addition, exemplified are compounds which zirconium as a central metal of the above-mentioned compounds is substituted by titanium or hafnium.

A co-catalyst containing aluminum is used as a component (B) in the catalyst used in the present invention. Examples of the co-catalyst containing aluminum include organic aluminum oxy compounds and organic aluminum compounds, and one kind of them may be used alone, or two or more kinds of them may be used in combination.

Examples of the organic aluminum oxy compounds include chain aluminoxanes each represented by a general formula (V)

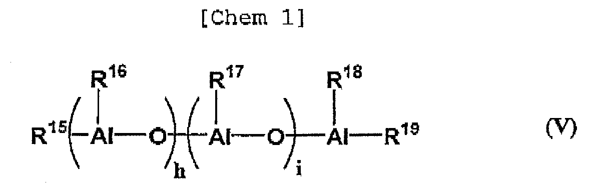

and cyclic aluminoxanes each represented by a general formula (VI).

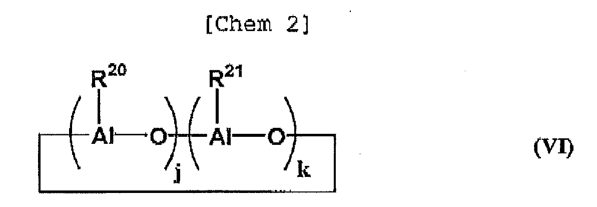

In the general formulae (V) and (VI), R¹⁵ to R²¹ each independently represent an alkyl group having 1 to 8 carbon atoms, h to k each represent a number of 0 to 50, and sums (h+i) and (j+k) are each equal to or larger than 1.

In the oxygen-containing organo-metallic compound represented by the general formulae (V) and (VI), examples of the alkyl group having 1 to 8 carbon atoms represented by R¹⁵ to R²¹ include a methyl group, an ethyl group, an n-propyl group, an isopropyl group, various butyl groups, various pentyl groups, various hexyl groups, various heptyl groups, and various octyl groups.
In addition, the value for each of h to k falls within the range of preferably 1 to 20, or particularly preferably 1 to 5.
Specific examples of the compound represented by the general formulae (V) and (VI) include a linear or cyclic tetramethyl dialmoxane, tetraisobutyl dialmoxane, methyl almoxane, ethyl almoxane, butyl almoxane, and isobutyl almoxane.

Examples of the organic aluminum compound used in the present invention include tri(n-alkyl)aluminum such as trimethylaluminum, triethylaluminum, tri(n-butyl)aluminum, tripropylaluminum, tripentylaluminum, trihexylaluminum, trioctylaluminum, and tridecylaluminum; tri branched-chain alkyl aluminum such as triisopropylaluminum, triisobutylaluminum, tri(sec-butyl)aluminum, tri(tert-butyl)aluminum, tri(2-methylbutyl)aluminum, tri(3-methylbutyl)aluminum, tri(2-methylpentyl)aluminum, tri(3-methylpentyl)aluminum, tri(4-methylpentyl)aluminum, tri(2-methylhexyl)aluminum, tri(3-methylhexyl)aluminum, and tri(2-ethylhexyl)aluminum; tricycloalkylaluminum such as tricyclohexylaluminum and tricyclooctylaluminum; triarylaluminum such as triphenylaluminum and tritolylaluminum; dialkylaluminum hydride such as diisopropylaluminum hydride and diisobutylaluminum hydride; alkenylaluminum such as isoprenylaluminum; alkylaluminum alkoxide such as isobutylaluminum methoxide, isobutylaluminum ethoxide, and isobutylaluminum isopropoxide; dialkylaluminum alkoxide such as dimethylaluminum methoxide, diethylaluminum ethoxide, and dibutylaluminum butoxide; alkylaluminum sesquialkoxide such as ethylaluminum sesquiethoxide and butylaluminum sesquibutoxide; partially alkoxylated alkylaluminum; alkylaluminum aryloxide such as diethylaluminum phenoxide, diethyl aluminum(2,6-di-t-butyl-4-methylphenoxide), ethylaluminum bis(2,6-di-t-butyl-4-methylphenoxide), diisobutylaluminum(2,6-di-t-butyl-4-methylphenoxide), and isobutylaluminum bis(2,6-di-t-butyl-4-methylphenoxide); dialkylaluminum halide such as dimethylaluminum chloride, diethylaluminum chloride, dibutylaluminum chloride, diethylaluminum bromide, and diisobutylaluminum chloride; alkylaluminum sesquihalide such as ethylaluminum sesquichloride, butylaluminum sesquichloride, and ethylaluminum sesquibromide; partially halogenated alkylaluminum such as alkylaluminum dihalide including ethylaluminum dichloride, propylaluminum dichloride, and butylaluminum dibromide; dialkylaluminum hydride such as diethylaluminum hydride and dibutylaluminum hydride; other partially hydrogenated alkylaluminum such as alkylaluminum dihydride including ethylaluminum dihydride and propylaluminum dihydride; and partially alkoxylated and halogenated alkylaluminum such as ethylaluminum ethoxychloride, butylaluminum buthoxychloride, and ethylaluminum ethoxybromide.

In general, an α-olefin having 3 to 14 carbon atoms is used as the α-olefin used in the present invention, and specific examples thereof include propylene, 1-butene, 3-methyl-1-butene, 4-methyl-1-butene, 4-phenyl-1-butene, 1-pentene, 3-methyl-1-pentene, 4-methyl-1-pentene, 3,3-dimethyl-1-pentene, 3,4-dimethyl-1-pentene, 4,4-dimethyl-1-pentene, 1-hexene, 4-methyl-1-hexene, 5-methyl-1-hexene, 6-phenyl-1-hexene, 1-octene, 1-decene, 7-dodecene, and 1-tetradecene. Of those, an α-olefin having 8 to 12 carbon atoms is preferable, and an α-olefin having 10 carbon atoms is particularly preferable.

In the method of producing an α-olefin oligomer composition of the present invention, the above catalyst is preferably used on condition that an aluminum concentration and a hydrogen pressure each fall within a specific numerical range.
The preferable ranges are, for example, as follows: an aluminum amount is 1.89×10⁻⁵ to 5.67×10⁻² mmol per mol of the α-olefin, and the hydrogen pressure is 1 to 50 kPa (G). The above aluminum amount corresponds to 0.1 to 300 mmol per liter of 1-decene when 1-decene is used as the α-olefin.

When the aluminum concentration does not satisfy the above requirement upon use of the co-catalyst containing aluminum, the following problems may arise. That is, when the aluminum concentration is less than 1.89×10⁻⁵ mol per mol of the α-olefin, the catalyst may exert no catalytic activity. Accordingly, the yield in which a target α-olefin oligomer is obtained reduces in some cases. On the other hand, when the aluminum concentration exceeds 5.67×10⁻² mol per mol of the α-olefin, the usage of Al is large, so hydrochloric acid, NaOH, or the like must be used in a large amount for decalcification. Accordingly, a disadvantageous effect is exerted on an environment, and the yield in which a target unsaturated hydrocarbon compound is obtained reduces in some cases. From the foregoing viewpoints, the aluminum concentration is preferably 9.45×10⁻⁵ to 3.78×10⁻² mol per mol of the α-olefin, or more preferably 1.51×10⁻⁴ to 1.89×10⁻² mol per mol of the α-olefin.

When the addition amount of hydrogen falls short of the above range, the activity of the catalyst reduces. When the amount outstrips the range, a saturated body of the α-olefin as a raw material is produced, so the yield in which the target α-olefin oligomer is obtained reduces in some cases. From the foregoing viewpoints, the addition amount of hydrogen is preferably 0.5 to 30 kPa (G), or more preferably 1 to 10 kPa (G).

The transition metal compound is used in an amount of typically 1.89×10⁻⁷ to 7.56×10⁻⁵ mol, preferably 7.56×10⁻⁷ to 5.67×10⁻⁵ mol, or more preferably 1.89×10⁻⁶ to 3.78×10⁻⁵ mol per mol of the α-olefin. When the amount is less than 1.89×10⁻⁷ mol, the catalyst may exert no catalytic activity. On the other hand, when the amount exceeds 7.56×10⁻⁵ mol, the amount in which the dimer of the α-olefin is produced increases, so the yield in which the target α-olefin oligomer is obtained reduces in some cases.
The above amount of the transition metal compound corresponds to typically 0.001 to 0.4 mmol, preferably 0.004 to 0.3 mmol, or more preferably 0.01 to 0.2 mmol per liter of 1-decene when 1-decene is used as the α-olefin.

The temperature at which the polymerization reaction is performed is typically 0 to 100°C, preferably 20 to 80°C, or more preferably 30 to 70°C. When the temperature is excessively low or excessively high, the catalyst system may exert no catalytic activity. In addition, when the temperature is excessively high, the dimer of the α-olefin is apt to be produced, so the yield in which the target α-olefin oligomer is obtained reduces in some cases.

When using a reaction solvent, examples thereof include aromatic hydrocarbons such as benzene, toluene, xylene, and ethylbenzene; alicyclic hydrocarbons such as cyclopentane, cyclohexane, and methylcyclohexane; aliphatic hydrocarbons such as pentane, hexane, heptane, and octane; and halogenated hydrocarbons such as chloroform and dichlorometane.

According to the present invention, an α-olefinoligomer composition suitable for a lubricating oil is obtained; to be specific, an α-olefin oligomer composition containing a trimer, a tetramer, and a pentamer each in a large amount is obtained. A ratio of the trimer, the tetramer, and the pentamer on the basis of the total amount of the α-olefin oligomer composition of the present invention is typically 30 mass% or more, preferably 40 mass% or more, or more preferably 50 mass% or more. In addition, the α-olefin oligomer composition is preferably an α-olefin oligomer composition satisfying the following relationships: A>B and A>C when a ratio of the trimer to the dimer on a mass basis is represented by A, a ratio of the tetramer to the trimer on a mass basis is represented by B, and a ratio of the pentamer to the tetramer on a mass basis is represented by C. The reason for the foregoing is as described below. That is, when a homogenous catalyst is used, α-olefin oligomers to be obtained are joint products, and an oligomer distribution varies depending on the kind of the catalyst and reaction conditions. When a reaction is performed with a catalyst using a transition metal compound free of any bulky substituent under reaction conditions within the ranges of the present invention, the dimer and a hexamer each tend to be produced in a large amount, so the yield in which the trimer, the tetramer, and the pentamer useful in lubricating oils are obtained reduces. On the other hand, when the catalyst used in the present invention is used, the ratio of the trimer to the dimer increases, so a ratio at which the trimer, the tetramer, and the pentamer are produced increases. The above relationships of A>B and A>C represent the tendency of each of the trimer, the tetramer, and the pentamer to be obtained in a large amount.

When the resultant α-olefin oligomer composition is used in a lubricating oil, a hydrogenated α-olefin oligomer composition may be produced by subjecting the composition to a hydrogenation treatment. In the hydrogenation treatment, a general hydrogenation catalyst such as Pd or Ni can be used, a temperature is typically 50 to 300°C, or preferably 60 to 200°C, and a hydrogen pressure is typically 0.1 to 10 MPa, or preferably 0.5 to 2 MPa.

The α-olefin oligomer composition and the hydrogenated α-olefin oligomer composition obtained by the above production methods can each be preferably used in the preparation of a lubricating oil composition. The applications of the lubricating oil composition are not particularly limited, and the composition can be used for lubricating an automobile, an aircraft, or any other industrial machine. The lubricating oil composition may be blended with a known additive as appropriate, and examples of the additive include a detergent dispersant, a viscosity index improver, an antioxidant, a corrosion inhibitor, an anti-wear agent, a friction adjustor, a pour point depressant, a rust inhibitor, a defoaming agent, and an extreme pressure agent.

The following production method as well as the above production method is included in the category of the method of producing an α-olefin oligomer of the present invention. It should be noted that the invention described below is referred to as "second invention of the present application."

The second invention of the present application relates to a method of producing an α-olefin oligomer including the step of polymerizing the molecules of an α-olefin by using a specific catalyst system while causing hydrogen to coexist with the α-olefin. It should be noted that the term "oligomer" as used in the second invention of the present application refers to a polymer obtained by the polymerization of a monomer or a composition of the polymer, and the oligomer may be substantially one kind of a specific polymer, or may be a mixture of two or more kinds (such as a dimer and a trimer).

A metallocene compound represented by a general formula (VII) is used in the catalyst system for use in the second invention of the present application.

(RC₅H₄)₂MX₂ (VII)

In the general formula (VII), R represents a hydrogen atom or a hydrocarbon group having 1 to 10 carbon atoms, M represents a transition metal element belonging to Group 4 of the periodic table, and X represents a covalent ligand or an ionic bond ligand.

In the general formula (VII), R preferably represents a hydrogen atom or a hydrocarbon group having 1 to 4 carbon atoms; specific examples of M include titanium, zirconium, and hafnium, and zirconium is preferable out of these elements; and specific examples of X include a hydrogen atom, a halogen atom, a hydrocarbon group having 1 to 20, or preferably 1 to 10 carbon atoms, an alkoxy group having 1 to 20, or preferably 1 to 10 carbon atoms, an amino group, a phosphorus-containing hydrocarbon group having 1 to 20, or preferably 1 to 12 carbon atoms (such as a diphenylphosphine group), a silicon-containing hydrocarbon group having 1 to 20, or preferably 1 to 12 carbon atoms (such as a trimethylsilyl group), and a boron compound containing a hydrocarbon group having 1 to 20, or preferably 1 to 12 carbon atoms or a halogen (such as B (C₆H₅)₄ or BF₄), and a group selected from a hydrogen atom, a halogen atom, a hydrocarbon group, and an alkoxy group is preferable out of these ligands.

Specific examples of the metallocene compound represented by the general formula (VII) include bis(cyclopentadienyl)zirconium dichloride, bis(methylcyclopentadienyl) zirconium dichloride, bis(ethylcyclopentadienyl)zirconium dichloride, bis(iso-propylcyclopentadienyl)zirconium dichloride, bis(n-propylcyclopentadienyl)zirconium dichloride, bis(n-butylcyclopentadienyl)zirconium dichloride, bis(t-butylcyclopentadienyl)zirconium dichloride, bis(thexylcyclopentadienyl)zirconium dichloride, bis(trimethylsilylcyclopentadienyl)zirconium dichloride, bis(trimethylsilylmethylcyclopentadienyl)zirconium dichloride, bis(cyclopentadienyl)zirconium chlorohydride, bis(cyclopentadienyl)methyl zirconium chloride, bis(cyclopentadienyl)ethyl zirconium chloride, bis(cyclopentadienyl)methoxy zirconium chloride, bis(cyclopentadienyl)phenyl zirconium chloride, bis(cyclopentadienyl)dimethyl zirconium, bis(cyclopentadienyl)diphenyl zirconium, bis(cyclopentadienyl)dineopentyl zirconium, bis(cyclopentadienyl)dihydrozirconium, and bis(cyclopentadienyl)dimethoxyzirconium. Further, exemplified are compounds which a chlorine atom of the above-mentioned compounds is substituted by a bromine atom, an iodine atom, a hydrogen atom, a methyl group, a phenyl group, or the like. In addition, exemplified are compounds which zirconium as a central metal of the above-mentioned compounds is substituted by titanium or hafnium.

A methylaluminoxane is used in the catalyst system for use in the second invention of the present application. The methylaluminoxane is not particularly limited, and a conventionally known methylaluminoxane can be used. Examples of such methylaluminoxane include a chain methylaluminoxane represented by a general formula (VIII) and a cyclic methylaluminoxane represented by a general formula (IX).

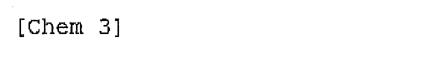

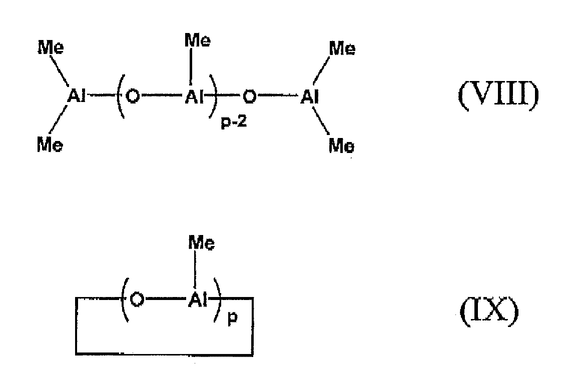

In the general formulae (VIII) and (IX), p represents a degree of polymerization which is typically 3 to 50, or preferably 7 to 40.

A method of producing the methylaluminoxane is, for example, a method involving bringing methylaluminum and a condensation agent such as water into contact with each other. However, an approach to the contact is not particularly limited, and it is sufficient that methylaluminum and the condensation agent be caused to react with each other in conformity with a known method. Examples of the method include: a method involving dissolving a methylaluminum compound in an organic solvent and bringing the solution into contact with water; a method involving originally adding a methylaluminum compound to the α-olefin at the time of the polymerization of the molecules of the α-olefin and adding water to the mixture later; a method involving causing crystal water in a metal salt or the like or water adsorbing to an inorganic or organic substance to react with a methylaluminum compound; and a method involving causing trimethylaluminum to react with tetramethyldialuminoxane and causing water to react with the reaction product. One kind of those methylaluminoxanes may be used, or two or more kinds of them may be used in combination.

The ratio (molar ratio) at which the metallocene compound and the methylaluminoxane in the catalyst system of the second invention of the present application are blended with each other "methylaluminoxane/metallocene compound" is typically 15 to 150, preferably 20 to 120, or more preferably 25 to 100. When the ratio is less than 15, the catalyst system may exert no catalytic activity. In addition, the dimer of the α-olefin is apt to be produced, so the yield in which a trimer or more is obtained reduces in some cases. On the other hand, when the ratio exceeds 150, the decalcification of the catalyst becomes incomplete in some cases.

In the second invention of the present application, an α-olefin having 3 to 14 carbon atoms is used as a monomer, and specific examples thereof include propylene, 1-butene, 3-methyl-1-butene, 4-methyl-1-butene, 4-phenyl-1-butene, 1-pentene, 3-methyl-1-pentene, 4-methyl-1-pentene, 3,3-dimethyl-1-pentene, 3,4-dimethyl-1-pentene, 4,4-dimethyl-1-pentene, 1-hexene, 4-methyl-1-hexene, 5-methyl-1-hexene, 6-phenyl-1-hexene, 1-octene, 1-decene, 1-dodecene, and 1-tetradecene. Of those, an α-olefin selected from 1-octene, 1-decene, and 1-dodecene is preferable, and 1-decene is particularly preferable.

In the second invention of the present application, the ratio at which the metallocene compound represented by the general formula (VII) and the α-olefin having 3 to 14 carbon atoms are blended with each other [metallocene compound (mmol)/α-olefin (L)] is typically 0.01 to 0.4, preferably 0.05 to 0.3, or more preferably 0.1 to 0.2. when the ratio is less than 0.01, the catalyst system may exert no catalytic activity. On the other hand, when the ratio exceeds 0.4, the dimer of the α-olefin is apt to be produced, so the yield in which an oligomer which is a trimer or more is obtained reduces, or the decalcification of the catalyst becomes incomplete in some cases.

In the second invention of the present application, the polymerization of the molecules of the above α-olefin having 3 to 14 carbon atoms is performed in the presence of hydrogen. The addition amount of hydrogen is typically 0.1 to 50 kPa, preferably 0.5 to 30 kPa, or more preferably 1 to 10 kPa. When the addition amount of hydrogen is smaller than 0.1 kPa, the catalytic activity of the catalyst system is not improved. When the amount is larger than 50 kPa, a saturated body of the α-olefin as a raw material is apt to be produced, so the yield in which the target α-olefin oligomer is obtained reduces in some cases.

In the second invention of the present application, a method for the polymerization reaction is not limited, and each of the following methods maybe employed: the reaction may be performed in the absence of any solvent, or may be performed in a solvent. When using a reaction solvent, examples thereof include aromatic hydrocarbons such as benzene, toluene, xylene, and ethylbenzene; alicyclic hydrocarbons such as cyclopentane, cyclohexane, and methylcyclohexane; aliphatic hydrocarbons such as pentane, hexane, heptane, and octane; and halogenated hydrocarbons such as chloroform and dichlorometane.

In the second invention of the present application, the temperature at which the polymerization reaction is performed is typically 0 to 100°C, preferably 20 to 80°C, or more preferably 30 to 70°C. When the temperature is excessively low or excessively high, the catalyst system may exert no catalytic activity. In addition, when the temperature is excessively high, the dimer of the α-olefin is apt to be produced, so the yield in which an oligomer which is a trimer or more is obtained reduces in some cases.

According to the second invention of the present application, α-olefin oligomers in which the selectivity of a trimer or more is 50% or more can be obtained, and such α-olefin oligomers can be preferably used in lubricating applications.

In the second invention of the present application, the α-olefin oligomer may be further treated as required. For example, when the thermal stability or oxidation stability of the α-olefin oligomer are improved, the following procedure is preferably adopted: a hydrogenated α-olefin oligomer is produced by subjecting the α-olefin oligomer to a hydrogenation treatment, and furthermore, the hydrogenated α-olefin oligomer is purified by distillation so that a purified hydrogenated α-olefin oligomer may be produced. The temperature at which the hydrogenation treatment is performed is typically 50 to 300°C, preferably 60 to 250°C, or more preferably 70 to 200°C, and a hydrogen pressure in the treatment is typically 0.1 to 10 MPa, preferably 0.5 to 2 MPa, or more preferably 0.7 to 1.5 MPa. A general hydrogenation catalyst containing Pd, Ni, or the like can be used in the hydrogenation treatment.

The temperature at which the distillation is performed is typically 200°C to 300°C, preferably 220 to 280°C, or more preferably 230 to 270°C, and the pressure at which the distillation is performed is typically 0.1 to 15 Pa, preferably 0.4 to 7 Pa, or more preferably 0.6 to 4 Pa.
A fraction having a kinematic viscosity at 100°C of 3 to 35 mm²/s obtained by the hydrogenation treatment and the distillation described above is particularly preferably used in lubricating applications.

The hydrogenated α-olefin oligomer obtained by the second invention of the present application has about 1 (typically 0.6 to 1.2, preferably 0.7 to 1.1, or more preferably 0.8 to 1.0) short-chain branch per molecule (it should be noted that, in the second invention of the present application, a methyl group, an ethyl group, and a propyl group are referred to as "short-chain branches"). Further, the short-chain branches in the hydrogenated α-olefin oligomer obtained by the second invention of the present application are mainly methyl groups, and the methyl groups account for typically 80 mol% or more, preferably 85 mol% or more, or more preferably 90 mol% or more of the short-chain branches. A hydrogenated α-olefin oligomer having such structure has the following characteristics: while the oligomer has a low viscosity, the oligomer is lost in a small amount by its evaporation.

### Examples

Next, the present invention is described in more detail by way of examples. However, the present invention is by no means limited by these examples.

In each of Examples 1 to 24, 36, 37 and Comparative Examples 1 to 3, an oligomer distribution and a degree of conversion were analyzed by gas chromatography. Measurement conditions are described below.
Column: HT-SIMDST (5 m×0.53 mm×0.17 µm)
Carrier flow rate: 40 cm/sec
Injection mode: Cool on-column injection
Injection/detection temperature: 440°C
Column temperature: 50 °C (held for 0. 1 minute), temperature increase at 20°C/min, 430°C (held for 15 minutes)
Injection amount: 0.5 µL
Sample concentration: A 1 mass% toluene solution (containing 1 mass% of a hexadecane internal standard)

A value for an oligomer selectivity was determined by representing ratios "trimer/dimer", "tetramer/trimer", and "pentamer/tetramer" by A, B, and C, respectively.

### [Example 1]

First, 250 mL of 1-decene were loaded into a glass container dried by heating and having an internal volume of 300 mL under a nitrogen atmosphere. Then, 1.2 mL of a 1 mol/L methylaluminoxane (MAO) were added to the container, and the temperature of the mixture was increased to 50°C. Next, 4 mL of a solution of bis(t-butylcyclopentadienyl) zirconium dichloride in toluene with its concentration adjusted to 10 mmol/L were added to the mixture, and the whole was subjected to a reaction at a hydrogen pressure of 5 kPa (G) and 50°C for 6 hours. In the experiment, an Al amount was 9.07×10⁻⁴ mol per mol of 1-decene, and a ratio (molar ratio) "aluminum/transition metal compound" was 30.
The reaction was stopped with 50 mL of 1 mass% dilute hydrochloric acid, and the reaction product was washed with 50 mL of deionized water twice so that a catalyst component might be decomposed and removed. The analysis of the resultant solution by gas chromatography showed that the selectivity of a dimer or more was as follows: the selectivities of a dimer, a trimer, a tetramer, a pentamer, and a hexamer or more were 47 mass%, 26 mass%, 11 mass%, 6 mass%, and 10 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 0.55, 0.42, and 0.36, respectively, so the relationships of A>B and A>C are satisfied.

### [Example 2]

A solution was prepared in the same manner as in Example 1 except that bis(trimethylsilylcyclopentadienyl)zirconium dichloride was used as a transition metal compound. The selectivity of a dimer or more was as follows: the selectivities of a dimer, a trimer, a tetramer, a pentamer, and a hexamer or more were 25 mass%, 24 mass%, 10 mass%, 6 mass%, and 35 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 0.96, 0.42, and 0.60, respectively, so the relationships of A>B and A>C are satisfied.

### [Comparative Example 1]

A solution was prepared in the same manner as in Example 1 except that bis (cyclopentadienyl) zirconium dichloride was used as a transition metal compound. The selectivity of a dimer or more was as follows: the selectivities of a dimer, a trimer, a tetramer, a pentamer, and a hexamer or more were 42 mass%, 11 mass%, 7 mass%, 5 mass%, and 35 mass%, respectively. In addition, the elemental analysis of the solution showed that the solution contained Cl, Al, and Zr at contents of less than 2 ppm by mass each, and was hence substantially free of any catalyst residue. In addition, the ratios A, B, and C are 0.26, 0.64, and 0.71, respectively, so the relationships of A<B and A<C are satisfied.
In Comparative Example 1, the dimer and the hexamer are each produced in a large amount, so the yield in which the trimer, the tetramer, and the pentamer useful in lubricating oils are obtained is low.

### [Example 36]

A solution was prepared in the same manner as in Example 1 except that bis(ethylcyclopentadienyl)zirconium dichloride was used as a transition metal compound. The selectivity of a dimer or more was as follows: the selectivities of a dimer, a trimer, a tetramer, a pentamer, and a hexamer or more were 28 mass%, 10 mass%, 7 mass%, 5 mass%, and 50 mass%, respectively. In addition, the elemental analysis of the solution showed that the solution contained Cl, Al, and Zr at contents of less than 2 ppm by mass each, and was hence substantially free of any catalyst residue. In addition, it can be found that the selectivity of each of the trimer, the tetramer, and the pentamer, is low because of the following reason: the ratios A, B, and Care 0.36, 0.70, and 0. 71, respectively, so the relationships of A>B and A>C are not satisfied.

### [Example 37]

A solution was prepared in the same manner as in Example 1 except that bis (isopropylcyclopentadienyl) zirconium dichloride was used as a transition metal compound. The selectivity of a dimer or more was as follows: the selectivities of a dimer, a trimer, a tetramer, a pentamer, and a hexamer or more were 28 mass%, 12 mass%, 8 mass%, 6 mass%, and 46 mass%, respectively. In addition, the elemental analysis of the solution showed that the solution contained Cl, Al, and Zr at contents of less than 2 ppm by mass each, and was hence substantially free of any catalyst residue. In addition, it can be found that the selectivity of each of the trimer, the tetramer, and the pentamer is low because of the following reason: the ratios A, B, and C are 0.43, 0.67, and 0.75, respectively, so the relationships of A>B and A>C are not satisfied.

### [Comparative Example 2]

A solution was prepared in the same manner as in Example 1 except that bis(trimethylsilylmethylcyclopentadienyl)zirconium dichloride was used as a transition metal compound. The selectivity of a dimer or more was as follows: the selectivities of a dimer, a trimer, a tetramer, a pentamer, and a hexamer or more were 13 mass%, 5 mass%, 4 mass%, 3 mass%, and 75 mass%, respectively. In addition, the elemental analysis of the solution showed that the solution contained Cl, Al, and Zr at contents of less than 2 ppm by mass each, and was hence substantially free of any catalyst residue. In addition, it can be found that the selectivity of each of the trimer, the tetramer, and the pentamer is low because of the following reason: the ratios A, B, and C are 0.38, 0.80, and 0.75, respectively, so the relationships of A>B and A>C are not satisfied.

[Table 1]

**Table 1**

| | | Example | | Comparative Example | Example | | Comparative Example |
|---|---|---|---|---|---|---|---|
| | | 1 | 2 | 1 | 36 | 37 | 2 |
| Transition metal compound | Cyclopentadienyl ligand | (CptBu)₂ | (CpTMS)₂ | Cp₂ | (CpEt)₂ | (CpiPr) ₂ | (CpTMSMe)₂ |
| | Usage (µmol) | 40 | 40 | 40 | 40 | 40 | 40 |
| | Amount per liter of 1-decene (mmol) | 0.16 | 0.16 | 0.16 | 0.16 | 0.16 | 0.16 |
| | Amount per mol of 1-decene (mol) | 3.03×10⁻⁵ | 3.03×.10⁻⁵ | 3.03×10⁻⁵ | 3.03×10⁻⁵ | 3.03×10⁻⁵ | 3.03×10⁻⁵ |
| Aluminum | Amount per liter of 1-decent (mmol) | 4.8 | 4.8 | 4.8 | 4.8 | 4.8 | 4.8 |
| | Amount per mol of 1-decene (mol) | 9.07×10⁻⁴ | 9.07×10⁻⁴ | 9.07×10⁻⁴ | 9.07×10⁻⁴ | 9.07×10⁻⁴ | 9.07×10⁻⁴ |
| Aluminum/transition metal compound (molar ratio) | | 30 | 30 | 30 | 30 | 30 | 30 |
| E₂ (kPaG) | | 5 | 5 | 5 | 5 | 5 | 5 |
| Temperature (°C) | | 50 | 50 | 50 | 50 | 50 | 50 |
| Degree of conversion (%) | | 90 | 50 | 94 | 50 | 58 | 28 |
| Production distribution (mass%) | Dimer | 47 | 25 | 42 | 28 | 28 | 13 |
| | Trimer | 26 | 24 | 11 | 10 | 12 | 5 |
| | Tetramer | 11 | 10 | 7 | 7 | 8 | 4 |
| | Pentamer | 4 | 6 | 5 | 5 | 6 | 3 |
| | Hexamer or more | 12 | 35 | 35 | 50 | 46 | 75 |
| A (trimer/dimer) | | 0.55 | 0.96 | 0.26 | 0.36 | 0.43 | 0.38 |
| B (tetramer/trimer) | | 0.42 | 0.42 | 0.64 | 0.70 | 0.67 | 0.80 |
| C (pentamer/tetramer) | | 0.36 | 0.60 | 0.71 | 0.71 | 0.75 | 0.75 |

### [Example 3]

A solution was prepared in the same manner as in Example 1 except that: the aluminum amount was changed to 1.81×10⁻² mol per mol of 1-decene; and the ratio (molar ratio) "aluminum/transition metal compound" was changed to 600. The selectivity of a dimer or more was as follows: the selectivities of a dimer, a trimer, tetramer, a pentamer, and a hexamer or more were 27 mass%, 28 mass%, 16 mass%, 10 mass%, and 20 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 1.04, 0.57, and 0.63, respectively, so the relationships of A>B and A>C are satisfied.

### [Example 4]

A solution was prepared in the same manner as in Example 1 except that: the aluminum amount was changed to 9.07×10⁻³ mol per mol of 1-decent; and the ratio (molar ratio) "aluminum/transition metal compound" was changed to 300. The selectivity of a dimer or more was as follows: the selectivities of a dimer, a trimer, a tetramer, a pentamer, and a hexamer or more were 54 mass%, 28 mass%, 9 mass%, 3 mass%, and 6 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 0.52, 0.32, and 0.33, respectively, so the relationships of A>B and A>C are satisfied.

### [Example 5]

A solution was prepared in the same manner as in Example 1 except that: the aluminum amount was changed to 3.02×10⁻³ mol per mol of 1-decene; and the ratio (molar ratio) "aluminum/transition metal compound" was changed to 100. The selectivity of a dimer or more was as follows: the selectivities of a dimer, a trimer, tetramer, a pentamer, and a hexamer or more were 44 mass%, 27 mass%, 11 mass%, 5 mass%, and 13 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 0.61, 0.41, and 0.45, respectively, so the relationships of A>B and A>C are satisfied.

### [Example 6]

A solution was prepared in the same manner as in Example 1 except that: the transition metal compound amount was changed to 2.27×10⁻⁵ mol per mol of 1-decent; the aluminum amount was changed to 9.07×10⁻⁴ mol per mol of 1-decene; and the ratio (molar ratio) "aluminum/transition metal compound" was changed to 40. The selectivity of a dimer or more was as follows: the selectivities of a dimer, a trimer, a tetramer, a pentamer, and a hexamer or more were 44 mass%, 25 mass%, 11 mass%, 6 mass%, and 9 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 0.57, 0.44, and 0.55, respectively, so the relationships of A>B and A>C are satisfied.

### [Example 7]

A solution was prepared in the same manner as in Example 1 except that: the transition metal compound amount was changed to 2.27×10⁻⁵ mol per mol of 1-decene; the aluminum amount was changed to 4.54×10⁻⁴ mol per mol of 1-decene; and the ratio (molar ratio) "aluminum/transition metal compound" was changed to 20. The selectivity of a dimer or more was as follows: the selectivities of a dimer, a trimer, a tetramer, a pentamer, and a hexamer or more were 37 mass%, 22 mass%, 11 mass%, 6mass%, and17mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 0.59, 0.50, and 0.55, respectively, so the relationships of A>B and A>C are satisfied.

[Table 2]

**Table 2**

| | | Example | | | | |
|---|---|---|---|---|---|---|
| | | 3 | 4 | 5 | 6 | 7 |
| Transition metal compound | Cyclopentadienyl ligand | (CptBu)₂ | (CptBu)₂ | (CptBu)₂ | (CptBu)₂ | (CptBu)₂ |
| | Usage (µmol) | 40 | 40 | 40 | 30 | 30 |
| | Amount per liter of 1-decene (mmol) | 0.16 | 0.16 | 0.16 | 0.12 | 0.12 |
| | Amount per mol of 1-decene (mol) | 3.03×10⁻⁵ | 3.03×10⁻⁵ | 3.03×10⁻⁵ | 2.27×10⁻⁵ | 2.27×10⁻⁵ |
| Aluminum | Amount per liter of (mmol) | 96 | 48 | 16 | 4.8 | 2.4 |
| | 1-decene Amount per mol of 1-decene | 1.81×10⁻² | 9.07×10⁻³ | 3.02×10⁻³ | 9.07×10⁻⁴ | 4.54×10⁻⁴ |
| Aluminum/transition metal compound (molar ratio) | | 600 | 300 | 100 | 40 | 20 |
| H₂ (kPaG) | | 5 | 5 | 5 | 5 | 5 |
| Temperature (°C) | | 50 | 50 | 50 | 50 | 50 |
| Degree of conversion (%) | | 96 | 95 | 94 | 99 | 98 |
| Production distribution (mass%) | Dimer | 27 | 54 | 44 | 44 | 37 |
| | Trimer | 28 | 28 | 27 | 25 | 22 |
| | Tetramer | 16 | 9 | 11 | 11 | 11 |
| | Pentamer | 10 | 3 | 5 | 6 | 6 |
| | Hexamer or more | 20 | 6 | 13 | 9 | 17 |
| A (trimer/dimer) | | 1.04 | 0.52 | 0.61 | 0.57 | 0.59 |
| B (tetramer/trimer) | | 0.57 | 0.32 | 0.41 | 0.44 | 0.50 |
| C (pentamer/tetramer) | | 0.63 | 0.33 | 0.45 | 0.55 | 0.55 |

### [Example 8]

A solution was prepared in the same manner as in Example 1 except that: the transition metal compound amount was changed to 2.27×10⁻⁵ mol per mol of 1-decene; the aluminum amount was changed to 4.54×10⁻⁴ mol per mol of 1-decene; the ratio (molar ratio) "aluminum/transition metal compound" was changed to 20; and an H₂ pressure was changed to 3 kPa (G). The selectivity of a dimer or more was as follows: the selectivities of a dimer, a trimer, a tetramer, a pentamer, and a hexamer or more were 42 mass%, 24 mass%, 11 mass%, 6 mass%, and 17 mass%, respectively. In addition, the elemental analysis of the solution showed that the solution contained Cl, Al, and Zr at contents of less than 2 ppm by weight each, and was hence substantially free of any catalyst residue. In addition, it can be found that an oligomer selectivity is high because of the following reason: the ratios A, B, and C are 0.57, 0.46, and 0.55 (C), respectively, so the relationships of A>B and A>C are satisfied.

### [Example 9]

A solution was prepared in the same manner as in Example 1 except that: the transition metal compound amount was changed to 1.89×10⁻⁵ mol per mol of 1-decene; the aluminum amount was changed to 4.73×10⁻⁴ mol per mol of 1-decene; and the ratio (molar ratio) "aluminum/transition metal compound" was changed to 25. The selectivity of a dimer or more was as follows: the selectivities of a dimer, a trimer, a tetramer, a pentamer, and a hexamer or more were 35 mass%, 23 mass%, 12 mass%, 7 mass%, and 23 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 0.66, 0.52, and 0.58, respectively, so the relationships of A>B and A>C are satisfied.

### [Example 10]

A solution was prepared in the same manner as in Example 1 except that: the transition metal compound amount was changed to 1.89×10⁻⁵ mol per mol of 1-decene; the aluminum amount was changed to 4.73×10⁻⁴ mol per mol of 1-decene; the ratio (molar ratio) "aluminum/transition metal compound" was changed to 25; and an H₂ pressure was changed to 10 kPa. The selectivity of a dimer or more was as follows: the selectivities of a dimer, a trimer, a tetramer, a pentamer, and a hexamer or more were 35 mass%, 23 mass%, 12 mass%, 7 mass%, and 23 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason; the ratios A, B, and C are 0.66, 0.52, and 0.58, respectively, so the relationships of A>B and A>C are satisfied.

### [Example 11]

A solution was prepared in the same manner as in Example 1 except that: the transition metal compound amount was changed to 1.89×10⁻⁵ mol per mol of 1-decene; the aluminum amount was changed to 4.73×10⁻⁴ mol per mol of 1-decene; and the ratio (molar ratio) "aluminum/transition metal compound" was changed to 25; and an H₂ pressure was changed to 3 kPa. The selectivity of a dimer or more was as follows: the selectivities of a dimer, a trimer, a tetramer, a pentamer, and a hexamer or more were 38 mass%, 25 mass%, 12 mass%, 7 mass%, and 18 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 0.66, 0.48, and 0.58, respectively, so the relationships of A>B and A>C are satisfied.

### [Example 12]

A solution was prepared in the same manner as in Example 1 except that: the transition metal compound amount was changed to 1.51×10⁻⁵ mol per mol of 1-decene; the aluminum amount was changed to 9.07×10⁻⁴ mol per mol of 1-decene; and the ratio (molar ratio) "aluminum/transition metal compound" was changed to 60. The selectivity of a dimer or more was as follows: the selectivities of a dimer, a trimer, a tetramer, a pentamer, and a hexamer or more were 37 mass%, 24 mass%, 12 mass%, 7 mass, and 18 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 0.65, 0.50, and 0.58, respectively, so the relationships of A>B and A>C are satisfied.

[Table 3]

**Table 3**

| | | Example | | | | |
|---|---|---|---|---|---|---|
| | | 8 | 9 | 10 | 11 | 12 |
| Transition metal compound | Cyclopentadienyl ligand | (CptBu)₂ | (CptBu)₂ | (CptBu)₂ | (CptBu)₂ | (CptBu)₂ |
| | Usage (µmol) | 30 | 25 | 25 | 25 | 20 |
| | Amount per liter of 1-decene (mmol) | 0.12 | 0.1 | 0.1 | 0.1 | 0.08 |
| | Amount per mol of 1-decene (mol) | 2.27×10⁻⁵ | 1.89×10⁻⁵ | 1.89×10⁻⁵ | 1.89×10⁻⁵ | 1.51×10⁻⁵ |
| Aluminum | Amount per liter of 1-decent (mmol) | 2.4 | 2.5 | 2.5 | 2.5 | 4.8 |
| | Amount per mol of 1-decene (mol) | 4.54×10⁻⁴ | 4.73×10⁻⁴ | 4.73×10⁻⁴ | 4.73×10⁻⁴ | 9.07×10⁻⁴ |
| Aluminum/transition metal compound (molar ratio) | | 20 | 25 | 25 | 25 | 60 |
| H₂ (kPaG) | | 3 | 5 | 10 | 3 | 5 |
| Temperature (°C) | | 50 | 50 | 50 | 50 | 50 |
| Degree of conversion (%) | | 98 | 97 | 98 | 88 | 98 |
| Production distribution (mass%) | Dimer | 42 | 35 | 35 | 38 | 37 |
| | Trimer | 24 | 23 | 23 | 25 | 24 |
| | Tetramer | 11 | 12 | 12 | 12 | 12 |
| | Pentamer | 6 | 7 | 7 | 7 | 7 |
| | Hexamer or more | 17 | 23 | 23 | 18 | 20 |
| A (trimer/dimer) | | 0.57 | 0.66 | 0.66 | 0.66 | 0.65 |
| B (tetramer/trimer) | | 0.46 | 0.52 | 0.52 | 0.48 | 0.50 |
| C (pentamer/tetramer) | | 0.55 | 0.58 | 0.58 | 0.58 | 0.58 |

### [Example 13]

A solution was prepared in the same manner as in Example 1 except that: the transition metal compound amount was changed to 1.51×10⁻⁵ mol per mol of 1-decent; the aluminum amount was changed to 6.05×10⁻⁴ mol per mol of 1-decene; and the ratio (molar ratio) "aluminum/transition metal compound" was changed to 40. The selectivity of a dimer or more was as follows: the selectivities of a dimer, a trimer, a tetramer, a pentamer, and a hexamer or more were 39 mass%, 25 mass%, 12 mass%, 7 mass%, and 16 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 0.64, 0.48, and 0.58, respectively, so the relationships of A>B and A>C are satisfied.

### [Example 14]

A solution was prepared in the same manner as in Example 1 except that: the transition metal compound amount was changed to 1.51×10⁻⁵ mol per mol of 1-decene; the aluminum amount was changed to 3.02×10⁻⁴ mol per mol of 1-decene; and the ratio (molar ratio) "aluminum/transition metal compound" was changed to 20. The selectivity of a dimer or more was as follows: the selectivities of a dimer, a trimer, a tetramer, a pentamer, and a hexamer or more were 31 mass%, 21mass%, 12mass%, 8 mass%, and25mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 0.68, 0.57, and 0.67, respectively, so the relationships of A>B and A>C are satisfied.

### [Example 15]

A solution was prepared in the same manner as in Example 1 except that: the transition metal compound amount was changed to 1.51×10⁻⁵ mol per mol of 1-decene; the aluminum amount was changed to 3.78×10⁻⁴ mol per mol of 1-decene; and the ratio (molar ratio) "aluminum/transition metal compound" was changed to 25. The selectivity of a dimer or more was as follows: the selectivities of a dimer, a trimer, a tetramer, a pentamer, and a hexamer or more were 35 mass%, 24 mass%, 12 mass%, 7 mass%, and 18 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 0.69, 0.50, and 0.58, respectively, so the relationships of A>B and A>C are satisfied.

### [Example 16]

A solution was prepared in the same manner as in Example 1 except that: the transition metal compound amount was changed to 1.13×10⁻⁵ mol per mol of 1-decene; the aluminum amount was changed to 3.40×10⁻⁴ mol per mol of 1-decene; and the ratio (molar ratio) "aluminum/transition metal compound" was changed to 30. The selectivity of a dimer or more was as follows: the selectivities of a dimer, a trimer, a tetramer, a pentamer, and a hexamer or more were 24 mass%, 21 mass%, 12 mass%, 8 mass%, and 21 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 0.88, 0.57, and 0.67, respectively, so the relationships of A>B and A>C are satisfied.

### [Example 17]

A solution was prepared in the same manner as in Example 1 except that: the transition metal compound amount was changed to 1.13×10⁻⁵ mol per mol of 1-decene; the aluminum amount was changed to 3.40×10⁻⁴ mol per mol of 1-decene; the ratio (molar ratio) "aluminum/transition metal compound" was changed to 30; and a reaction temperature was changed to 40°C. The selectivity of a dimer or more was as follows: the selectivities of a dimer, a trimer, a tetramer, a pentamer, and a hexamer or more were 22 mass%, 19 mass%, 11 mass%, 8 mass%, and 35 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 0.86, 0.58, and 0.73, respectively, so the relationships of A>B and A>C are satisfied.

### [Example 18]

A solution was prepared in the same manner as in Example 1 except that: the transition metal compound amount was changed to 3.78×10⁻⁶ mol per mol of 1-decene; the aluminum amount was changed to 4.54×10⁻⁴ mol per mol of 1-decene; the ratio (molar ratio) "aluminum/transition metal compound" was changed to 120; and the reaction temperature was changed to 50°C. The selectivity of a dimer or more was as follows: the selectivities of a dimer, a trimer, a tetramer, a pentamer, and a hexamer or more were 29 mass%, 26 mass%, 14 mass%, 8 mass%, and 23 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 0.90, 0.54, and 0.57, respectively, so the relationships of A>B and A>C are satisfied.

### [Comparative Example 3]

First, 100 mL of 1-decene and 100 mL of toluene were loaded into a stainless steel autoclave dried by heating and having an internal volume of 1 L. Then, 20 mL of a 3.3 mol/L MAO were added to the autoclave, and the temperature of the mixture was increased to 60°C. Next, 40 mL of a solution ofbis (cyclopentadienyl) zirconium dichloride in toluene with its concentration adjusted to 6.2 mmol/L were added to the mixture, and the whole was subjected to a reaction at 60°C for 1 hour. In the experiment, an Al amount was 0.125 mol per mol of α-olefin, and a ratio (molar ratio) "aluminum/transition metal compound" was 266.
The reaction was stopped with 50 mL of 1 mass% dilute hydrochloric acid, and the reaction product was washed with 50 mL of deionized water twice so that a catalyst component might be decomposed and removed. The analysis of the resultant solution by gas chromatography showed that the selectivity of a dimer or more was as follows: the selectivities of a dimer, a trimer, a tetramer, a pentamer, and a hexamer or more were 60 mass%, 23 mass%, 9 mass%, 3 mass%, and 5 mass%, respectively. In addition, the elemental analysis of the solution showed that the solution contained Cl, Al, and Zr at contents of 5 ppm, 15 ppm, and 6 ppm by mass each, and was hence not completely free of any catalyst residue.

[Table 4]

**Table 4**

| | | Example | | | | | | Comparative Example |
|---|---|---|---|---|---|---|---|---|
| | | 13 | 14 | 15 | 16 | 17 | 18 | 3 |
| Transition metal compound | Cyclopentadienyl ligand | (CptBu) ₂ | (CptBu)₂ | (CptBu)₂ | (CptBu)₂ | (CptBu)₂ | (CptBu)₂ | Cp₂ |
| | Usage (µmol) | 20 | 20 | 20 | 15 | 15 | 5 | 248 |
| | Amount per liter of 1-decene (mmol) | 0.08 | 0.08 | 0.08 | 0.06 | 0.06 | 0.02 | 2.48 |
| | Amount per mol of 1-decene (mol) | 1.51×10⁻⁵ | 1.51×10⁻⁵ | 1.51×14⁻⁵ | 1.13×10⁻⁵ | 1.13×10⁻⁵ | 3.78×10⁻⁶ | 4.69×10⁻⁴ |
| Aluminum | Amount per liter of 1-decene (mmol) | 3.2 | 1.6 | 2 | 1.8 | 1.8 | 2.4 | 660 |
| | Amount per mol of 1-decene (mol) | 6.05×10⁻⁴ | 3.02×10⁻⁴ | 3.78×10⁻⁴ | 3.40×10⁻⁴ | 3.40×10⁻⁴ | 4.54×10⁻⁴ | 0.125 |
| Aluminum/transition metal compound (molar ratio) | | 40 | 20 | 25 | 30 | 30 | 120 | 266 |
| H₂ (kPaG) | | 5 | 5 | 3 | 5 | 5 | 5 | 0 |
| Temperature (°C) | | 50 | 50 | 50 | 50 | 40 | 50 | 60 |
| Degree of conversion (%) | | 72 | 70 | 85 | 62 | 90 | 85 | 94 |
| Production distribution (mass%) | Dimer | 39 | 31 | 35 | 24 | 22 | 29 | 60 |
| | Trimer | 25 | 21 | 24 | 21 | 19 | 26 | 23 |
| | Tetramer | 12 | 12 | 12 | 12 | 11 | 14 | 9 |
| | Pentamer | 7 | 8 | 7 | 8 | 8 | 8 | 3 |
| | Hexamer or more | 17 | 28 | 22 | 35 | 40 | 23 | 5 |
| A (trimer/dimer) | | 0.64 | 0.68 | 0.69 | 0.88 | 0.86 | 0.90 | 0.38 |
| B (tetramer/trimer) | | 0.48 | 0.57 | 0.50 | 0.57 | 0.58 | 0.54 | 0.39 |
| C (pentamer/tetramer) | | 0.58 | 0.67 | 0.58 | 0.67 | 0.73 | 0.57 | 0.33 |

### [Example 19]

A solution was prepared in the same manner as in Example 1 except that: bis((3-methylpentane-3-yl)cyclopentadienyl)zirconium dichloride was used as a transition metal compound and its amount was changed to 7.55×10⁻⁶ mol per mol of 1-decene; the aluminum amount was changed to 7.55×10⁻⁴ mol per mol of 1-decene; the ratio (molar ratio) "aluminum/transition metal compound" was changed to 100; and the reaction temperature was changed to 40°C. The selectivity of a dimer or more was as follows: the selectivities of a dimer, a trimer, a tetramer, a pentamer, and a hexamer or more were 15 mass%, 27 mass%, 19 mass%, 12 mass%, and 28 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 1.8, 0.7, and 0.63, respectively, so the relationships of A>B and A>C are satisfied.

### [Example 20]

A solution was prepared in the same manner as in Example 19 except that: the transition metal compound amount was changed to 1.89×10⁻⁵ mol per mol of 1-decene; the aluminum amount was changed to 1.89×10⁻³ mol per mol of 1-decene; the ratio (molar ratio) "aluminum/transition metal compound" was changed to 100; and the reaction temperature was changed to 50 °C. The selectivity of a dimer or more was as follows: the selectivities of a dimer, a trimer, a tetramer, a pentamer, and a hexamer or more were 31 mass%, 34 mass%, 16 mass%, 7 mass%, and 12 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 1.1, 0.47, and 0.44, respectively, so the relationships of A>B and A>C are satisfied.

### [Example 21]

A solution was prepared in the same manner as in Example 19 except that: the transition metal compound amount was changed to 1.89×10⁻⁵ mol per mol of 1-decene; the aluminum amount was changed to 5.66×10⁻³ mol per mol of 1-decene; the ratio (molar ratio) "aluminum/transition metal compound" was changed to 300; and the reaction temperature was changed to 35°C, The selectivity of a dimer or more was as follows: the selectivities of a dimer, a trimer, a tetramer, a pentamer, and a hexamer or more were 20 mass%, 29 mass%, 17 mass%, 10 mass%, and 23 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 1.4, 0.61, and 0.59, respectively, so the relationships of A>B and A>C are satisfied.

### [Example 22]

A solution was prepared in the same manner as in Example 19 except that: the transition metal compound amount was changed to 7.55×10⁻⁵ mol per mol of 1-decene; the aluminum amount was changed to 7.55×10⁻³ mol per mol of 1-decene; the ratio (molar ratio) "aluminum/transition metal compound" was changed to 1,000; and the reaction temperature was changed to 30 °C. The selectivity of a dimer or more was as follows: the selectivities of a dimer, a trimer, a tetramer, a pentamer, and a hexamer or more were 11 mass%, 23 mass%, 17 mass%, 12 mass%, and 36 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 2.1, 0.74, and 0.71, respectively, so the relationships of A>B and A>C are satisfied.

### [Example 23]

A solution was prepared in the same manner as in Example 19 except that: the transition metal compound amount was changed to 3.77×10⁻⁵ mol per mol of 1-decene; the aluminum amount was changed to 7.55×10⁻⁴ mol per mol of 1-decene; the ratio (molar ratio) "aluminum/transition metal compound" was changed to 20; and the reaction temperature was changed to 40°C. The selectivity of a dimer or more was as follows: the selectivities of a dimer, a trimer, a tetramer, a pentamer, and a hexamer or more were 23 mass%, 28 mass%, 17 mass%, 11 mass%, and 21 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 1.2, 0.61, and 0.65, respectively, so the relationships of A>B and A>C are satisfied.

### [Example 24]

A solution was prepared in the same manner as in Example 19 except that: the transition metal compound amount was changed to 3.77×10⁻⁶ mol per mol of 1-decene; the aluminum amount was changed to 1.51×10⁻³ mol per mol of 1-decene; the ratio (molar ratio) "aluminum/transition metal compound" was changed to 400; and the reaction temperature was changed to 40 °C. The selectivity of a dimer or more was as follows: the selectivities of a dimer, a trimer, a tetramer, a pentamer, and a hexamer or more were 27 mass%, 28 mass%, 17 mass%, 10 mass%, and 17 mass%, respectively. In addition, the elemental analysis of the solution showed that the solution contained Cl, Al, and Zr at contents of less than 2 ppm by mass each, and was hence substantially free of any catalyst residue. In addition, it can be found that an oligomer selectivity is high because of the following reason: the ratios A, B, and C are 1.0, 0.61, and 0.59, respectively, so the relationships of A>B and A>C are satisfied.

[Table 5]

**Table 5**

| | | Example | | | | | |
|---|---|---|---|---|---|---|---|
| | | 19 | 20 | 21 | 22 | 23 | 24 |
| Transition metal compound | Cyclopentadienyl ligand | (CpMeCEt₂)₂ | (CpMeCEt₂)₂ | (CpMeCEt₂)₂ | (CpMeCEt₂)₂ | (CpMeCEt₂)₂ | (CpMeCEt₂)₂ |
| | usage (µmol) | 10 | 25 | 25 | 10 | 50 | |
| | per liter of 1-decene (mmol) | 0.04 | 0.1 | 0.1 | 0.04 | 0.2 | 0.02 |
| | Amount per mol of 1-decene (mol) | 7.55×10⁻⁶ | 1.89×10⁻⁵ | 1.89×10⁻⁵ | 7.55×10⁻⁶ | 3.77×10⁻⁵ | 3.77×10⁻⁶ |
| Aluminum | Amount per liter of 1-decene (mmol) | 4 | 10 | 30 | 40 | 4 | 8 |
| | Amount per mol of 1-decene (mol) | 7.55×10⁻⁴ | 1.89×10⁻³ | 5.66×10⁻³ | 7.55×10⁻³ | 7.55×10⁻⁴ | 1.51×10⁻³ |
| Aluminum/transition metal compound (molar ratio) | | 100 | 100 | 300 | 1,000 | 20 | 400 |
| H₂ (kPaG) | | 5 | 1 | 1 | 1 | 5 | 5 |
| Temperature (°C) | | 40 | 50 | 35 | 30 | 40 | 40 |
| Degree of conversion (%) | | 13 | 85 | 96 | 79 | 81 | 89 |
| Production distribution (mass%) | Dimer | 15 | 31 | 20 | 11 | 23 | 27 |
| | Trimer | 27 | 34 | 29 | 23 | 28 | 28 |
| | Tetramer | 19 | 16 | 17 | 17 | 17 | 17 |
| | Pentamer | 12 | 7 | 10 | 12 | 11 | 10 |
| | Hexamer or more | 27 | 12 | 23 | 36 | 21 | 18 |
| A (trimer/dimer) | | 1.8 | 1.1 | 1.4 | 2.1 | 1.2 | 1.0 |
| B (tetramer/trimer) | | 0.70 | 0.47 | 0.61 | 0.74 | 0.61 | 0.61 |
| C (pentamer/tetramer) | | 0.63 | 0.44 | 0.59 | 0.71 | 0.65 | 0.59 |

In each of Examples 25 to 30 and Comparative Examples 4 to 6, an oligomer yield, a dimer selectivity, and the like were analyzed by gas chromatography, and the amount of a catalyst residue was determined by elemental analysis.
In each of Examples 31 to 35 and Comparative Examples 7 to 13, the amount of each component in the entire oligomers was examined by analyzing a carbon distribution by gas chromatography. In addition, an average degree of polymerization was determined by gel permeation chromatography (GPC) measurement [average degree of polymerization: number-average molecular weight/molecular weight of a monomer in terms of polystyrene (PS) (average of values obtained by dividing molecular weights corresponding to a trimer, a tetramer, and a pentamer by 3, 4, and 5, respectively)] . In addition, the number of short-chain branches (short-chain branches/1,000 carbon atoms), the average number of short-chain branches per molecule of an oligomer (average degree of polymerization×number of carbon atoms of the monomerxnumber of short-chain branches/1,000), and a branching group ratio were measured by ¹³C-NMR (CDCl₃) measurement. A kinematic viscosity at 40°C and a kinematic viscosity at 100°C were measured in conformity with JIS K 2283. A viscosity index was measured in conformity with JIS K 2283. A pour point was measured in conformity with JIS K 2269. A flash point was measured in conformity with JIS K 2265 (Cleveland open-cup). With regard to an amount of evaporation (Noack), the amount in which a base oil was lost after 1 hour of its evaporation at 250°C was measured in conformity with ASTM D5800.

Details about measurement conditions are described below.

[Gas chromatography measurement]
Column: HT-SIMDST (5 m×0.53 mm×0.17 µm)
Carrier flow rate: 40 cm/sec
Injection mode: Cool on-column injection
Injection/detection temperature: 440°C
Column temperature: 50 °C (held for 0.1 minute), temperature increase at 20°C/min, 430°C (held for 15 minutes)
Injection amount: 0.5 µL
Sample concentration: A 1 mass% toluene solution (containing 1 mass% of a hexadecane internal standard)
[GPC measurement]
GPC column: Columns of TOSOH TSK-GEL MULTIPORE HXL-M (2 columns) and Shodex KF801 (1 column)
Solvent: THF
Temperature: 40°C
Flow velocity: 1.0 mL/min
Injection concentration: 0.1 mass%
Injection amount: 100 µl
Molecular weight conversion: Polystyrene (PS) conversion Detector: RI

[¹³C-NMR measurement]
Measuring machine: A LAMBDA 500 manufactured by JEOL
Sample solvent: CDCl₃
Measurement conditions: A 30° pulse, pulse repetition time=10 seconds, a total of 1,000 times, 26°C
Analysis: A peak in the range of 15 to 20.8 ppm is identified as a methyl group, a peak in the range of 14.5 to 15 ppm is identified as the methyl carbon atom of a propyl group, and a peak in the range of 10.3 to 13.5 ppm, is identified as the methyl carbon atom of an ethyl group.
Number of short-chain branches (short-chain branches/1, 000 carbon atoms) : Sum of area intensities of methyl group carbon atoms (methyl group+ethyl group+propyl group)÷(all area intensities in range of 10.3 to 46 ppm)×1,000

### [Example 25]

First, 2.5 L of 1-decene that had been deaerated by nitrogen bubbling and dehydrated and 2.5 L of toluene that had been similarly deaerated and dehydrated were loaded into a stainless steel autoclave replaced with nitrogen and having an internal volume of 5 L. After that, the temperature of the mixture was increased to 65°C, and 40 ml of a solution of a methylaluminoxane in toluene with its concentration adjusted to 1.0 mol/L were added to the mixture.
Next, 10 mL of a solution of bis (cyclopentadienyl) zirconium dichloride in toluene with its concentration adjusted to 40 mmol/L were added to the resultant mixture, and the whole was subjected to a reaction at 65°C for 3 hours while 5 kPa of hydrogen were continuously supplied and the whole was stirred.
In the above reaction, the metallocene compound was blended with 1-decene at a ratio of 0.16 mmol per liter of 1-decene, and a ratio (molar ratio) "methylaluminoxane/metallocene compound" was 100. The reaction was stopped with 500 mL of 1% dilute hydrochloric acid, and the reaction product was washed with 100 mL of deionized water twice so that a catalyst component might be decomposed and removed. The analysis of the resultant solution by gas chromatography showed that an oligomer yield was 86% and the selectivity of a dimer or more was as follows: the selectivities of a dimer, a trimer, a tetramer, a pentamer, and a hexamer or more were 49%, 17%, 10%, 6%, and 18%, respectively. In addition, the elemental analysis of the solution showed that the solution contained Cl, Al, and Zr at contents of less than 2 ppm by mass each, and was hence substantially free of any catalyst residue.

### [Example 26]

First, 2.5 L of 1-decene that had been deaerated by nitrogen bubbling and dehydrated were loaded into a stainless steel autoclave replaced with nitrogen and having an internal volume of 5 L. After that, the temperature of the mixture was increased to 50°C, and 12 ml of a solution of a methylaluminoxane in toluene with its concentration adjusted to 1.0 mol/L were added to the mixture.
Next, 10 mL of a solution of bis (cyclopentadienyl) zirconium, dichloride in toluene with its concentration adjusted to 40 mmol/L were added to the resultant mixture, and the whole was subjected to a reaction at 50°C for 7 hours while 5 kPa of hydrogen were continuously supplied and the whole was stirred.
In the above reaction, the metallocene compound was blended with 1-decene at a ratio of 0.16 mmol per liter of 1-decene, and a ratio (molar ratio) "methylaluminoxane/metallocene compound" was 30. The reaction was stopped with 500 mL of 1% dilute hydrochloric acid, and the reaction product was washed with 100 mL of deionized water twice so that a catalyst component might be decomposed and removed. The analysis of the resultant solution by gas chromatography showed that an oligomer yield was 94% and the selectivity of a dimer or more was as follows: the selectivities of a dimer, a trimer, a tetramer, a pentamer, and a hexamer or more were 42%, 11%, 7%, 5%, and 35%, respectively. In addition, the elemental analysis of the solution showed that the solution contained Cl, Al, and Zr at contents of less than 2 ppm by mass each, and was hence substantially free of any catalyst residue.

### [Example 27]

First, 250 mL of 1-decene that had been deaerated by nitrogen bubbling and dehydrated were loaded into a stainless steel autoclave replaced with nitrogen and having an internal volume of 1 L. After that, the temperature of the mixture was increased to 50°C, and 1.8 ml of a solution of a methylaluminoxane in toluene with its concentration adjusted to 1.0 mol/L were added to the mixture.
Next, 1 mL of a solution of bis(cyclopentadienyl)zirconium dichloride in toluene with its concentration adjusted to 40 mmol/L was added to the resultant mixture, the temperature of the mixture was increased to 50°C, and the whole was subjected to a reaction at 50 °C for 2 hours while 5 kPa of hydrogen were continuously supplied and the whole was stirred.
In the above reaction, the metallocene compound was blended with 1-decene at a ratio of 0.16 mmol per liter of 1-decene, and a ratio (molar ratio) "methylaluminoxane/metallocene compound" was 45. The reaction was stopped with 50 mL of 1% dilute hydrochloric acid, and the reaction product was washed with 10 mL of deionized water twice so that a catalyst component might be decomposed and removed. The analysis of the resultant solution by gas chromatography showed that an oligomer yield was 87% and the selectivity of a dimer or more was as follows: the selectivities of a dimer, a trimer, a tetramer, a pentamer, and a hexamer or more were 32%, 11%, 7%, 5%, and 45%, respectively. In addition, the elemental analysis of the solution showed that the solution contained Cl, Al, and Zr at contents of less than 2 ppm by mass each, and was hence substantially free of any catalyst residue.

### [Example 28]

First, 250 mL of 1-decene that had been deaerated by nitrogen bubbling and dehydrated were loaded into a stainless steel autoclave replaced with nitrogen and having an internal volume of 1 L. After that, the temperature of the mixture was increased to 50°C, and 0.6 ml of a solution of a methylaluminoxane in toluene with its concentration adjusted to 1.0 mol/L were added to the mixture.
Next, 1 mL of a solution of bis (t-butylcyclopentadienyl) zirconium dichloride in toluene with its concentration adjusted to 25 mmol/L was added to the resultant mixture, and the whole was subjected to a reaction at 50°C for 5 hours while 2.5 kPa of hydrogen were continuously supplied and the whole was stirred.
In the above reaction, the [metallocene compound was blended with 1-decene at a ratio of 0.10 mmol per liter of 1-decene, and a ratio (molar ratio) "methylaluminoxane/metallocene compound" was 24. The reaction was stopped with 50 mL of 1% dilute hydrochloric acid, and the reaction product was washed with 10 mL of deionized water twice so that a catalyst component might be decomposed and removed. The analysis of the resultant solution by gas chromatography showed that an oligomer yield was 92% and the selectivity of a dimer or more was as follows: the selectivities of a dimer, a trimer, a tetramer, a pentamer, and a hexamer or more were 42%, 24%, 12%, 7%, and 15%, respectively. In addition, the elemental analysis of the solution showed that the solution contained Cl, Al, and Zr at contents of less than 2 ppm by mass each, and was hence substantially free of any catalyst residue.

### [Example 29]

A reaction was performed in the same manner as in Example 27 except that: bis(ethylcyclopentadienyl)zirconium dichloride was used instead of bis (cyclopentadienyl) zirconium dichloride; and 1.2 ml of a solution of a methylaluminoxane in toluene with its concentration adjusted to 1.0 mol/L were used.
In the above reaction, the [metallocene compound was blended with 1-decene at a ratio of 0.16 mmol per liter of 1-decene, and a ratio (molar ratio) "methylaluminoxane/metallocene compound" was 30. The reaction was stopped with 50 mL of 1% dilute hydrochloric acid, and the reaction product was washed with 10 mL of deionized water twice so that a catalyst component might be decomposed and removed. The analysis of the resultant solution by gas chromatography showed that an oligomer yield was 79% and the selectivity of a dimer or more was as follows: the selectivities of a dimer, a trimer, a tetramer, a pentamer, and a hexamer or more were 28%, 10%, 7%, 5%, and 50%, respectively. In addition, the elemental analysis of the solution showed that the solution contained Cl, Al, and Zr at contents of less than 2 ppm by mass each, and was hence substantially free of any catalyst residue.

### [Example 30]

A reaction was performed in the same manner as in Example 29 except that: bis(iso-propylcyclopentadienyl)zirconium dichloride was used instead of bis(ethylcyclopentadienyl) zirconium dichloride.
In the above reaction, the metallocene compound was blended with 1-decene at a ratio of 0.16 mmol per liter of 1-decene, and a ratio (molar ratio) "methylaluminoxane/metallocene compound" was 30. The reaction was stopped with 50 mL of 1% dilute hydrochloric acid, and the reaction, product was washed with 10 mL of deionized water twice so that a catalyst component might be decomposed and removed. The analysis of the resultant solution by gas chromatography showed that an oligomer yield was 88% and the selectivity of a dimer or more was as follows: the selectivities of a dimer, a trimer, a tetramer, a pentamer, and a hexamer or more were 28%, 12%, 8%, 6%, and 46%, respectively. In addition, the elemental analysis of the solution showed that the solution contained Cl, Al, and Zr at contents of less than 2 ppm by mass each, and was hence substantially free of any catalyst residue.

### [Comparative Example 4]

First, 100 mL of 1-decene that had been deaerated by nitrogen bubbling and dehydrated and 100 L of toluene that had been similarly deaerated and dehydrated were loaded into a stainless steel autoclave replaced with nitrogen and having an internal volume of 1 L. After that, the temperature of the mixture was increased to 60°C, and 20 ml of a solution of a methylaluminoxane in toluene with its concentration adjusted to 3.3 mol/L were added to the mixture.
Next, 40 mL of a solution of bis (cyclopentadienyl) zirconium dichloride in toluene with its concentration adjusted to 6.2 mmol/L were added to the resultant mixture, and the whole was subjected to a reaction at 60 °C for 1 hour and the whole was stirred.
In the above reaction, the metallocene compound was blended with 1-decene at a ratio of 2.5 mmol per liter of 1-decene, and a ratio (molar ratio) "methylaluminoxane/metallocene compound" was 266. The reaction was stopped with 50 mL of 1% dilute hydrochloric acid, and the reaction product was washed with 10 mL of deionized water twice. A precipitate was produced near an interface between water and oil at the time of the decalcification of the catalyst in a large amount as compared to Examples 25 to 30. The elemental analysis of the resultant solution after water washing showed that the solution contained Cl, Al, and Zr at contents of 5 ppm by mass, 15 ppm by mass, and 6 ppm by mass, respectively, so catalyst residues could not be completely removed. An oligomer yield was 94%, and the selectivity of a dimer or more was as follow: the selectivities of a dimer, a trimer, a tetramer, a pentamer, and a hexamer or more were 60%, 23%, 9%, 3%, and 5%, respectively.

### [Comparative Example 5]

First, 400 mL of 1-decene that had been deaerated by nitrogen bubbling and dehydrated was loaded into a stainless steel autoclave replaced with nitrogen and having an internal volume of 1 L. After that, the temperature of the mixture was increased to 50°C, and 2 ml of a solution of a methylaluminoxane in toluene with its concentration adjusted to 1.0 mol/L were added to the mixture.
Next, 5 mL of a solution of bis(cyclopentadienyl) zirconium dichloride in toluene with its concentration adjusted to 40 mmol/L were added to the resultant mixture, and the whole was subjected to a reaction at 50°C for 5 hours while 100 kPa of hydrogen were continuously supplied and the whole was stirred.
In the above reaction, the metallocene compound was blended with 1-decene at a ratio of 0.5 mmol per liter of 1-decene, and a ratio (molar ratio) "methylaluminoxane/metallocene compound" was 10. The reaction was stopped with 50 mL of 1% dilute hydrochloric acid, and the reaction product was washed with 10 mL of deionized water twice so that a catalyst component might be decomposed and removed. The analysis of the resultant solution by gas chromatography showed that an oligomer yield was 90% and the selectivity of a dimer or more was as follows: the selectivities of a dimer, a trimer, a tetramer, a pentamer, and a hexamer or more were 77%, 15%, 5%, 2%, and 1%, respectively. In addition, the elemental analysis of the solution showed that the solution contained Cl, Al, and Zr at contents of less than 2 ppm by mass each, and was hence substantially free of any catalyst residue.

### [Comparative Example 6]

A solution was prepared in the same manner as in Example 28 except that H₂ was not introduced. In the reaction, the metallocene compound was blended with 1-decene at a ratio of 0.10 mmol per liter of 1-decene, and a ratio (molar ratio) "methylaluminoxane/metallocene compound" was 24. The reaction was stopped with 50 mL of 1% dilute hydrochloric acid, and the reaction product was washed with 10 mL of deionized water twice so that a catalyst component might be decomposed and removed. The analysis of the resultant solution by gas chromatography showed that an oligomer yield was extremely low, i.e. 25% and the selectivity of a dimer or more was as follows: the selectivities of a dimer, a trimer, a tetramer, a pentamer, and a hexamer or more were 46%, 21%, 9%, 5%, and 19%, respectively. In addition, the elemental analysis of the solution showed that the solution contained Cl, Al, and Zr at contents of less than 2 ppm by mass each, and was hence substantially free of any catalyst residue.

Tables 6 and 7 show the main production conditions of Examples 25 to 30 and Comparative Examples 4 to 6, and the results of the analysis of the resultant α-olefin oligomers.

[Table 6]

**Table 6**

| | | | Example | | | | | | Comparative Example | | |
|---|---|---|---|---|---|---|---|---|---|---|---|
| | | | 25 | 26 | 27 | 28 | 29 | 30 | 4 | 5 | 6 |
| (RC₅H₄)₂ZrCl₂ | (C₅H₅)₂ ZrCl₂ | mmol | 0.4 | 0.4 | 0.04 | - | - | - | 0.248 | 0.2 | - |
| | (tBuC₅H₄)₂ZrCl₂ | mmol | - | - | - | 0.025 | - | - | - | - | 0.025 |
| | (EtC₅H₄) ZrCl₂ | mmol | - | - | - | - | 0.04 | - | - | - | - |
| | (iPrC₅H₄) ZrCl₂ | mmol | - | - | - | - | - | 0.04 | - | - | - |
| MAO (methylaluminoxane) | | mmol | 40 | 12 | 1. 8 | 0.6 | 1.2 | 1.2 | 66 | 2 | 0.6 |
| 1-decene | | L | 2.5 | 2.5 | 0.25 | 0.25 | 0.25 | 0.25 | 0.1 | 0.4 | 0.25 |
| H₂ | | kPa | 5 | 5 | 5 | 2.5 | 5 | 5 | - | 100 | - |
| Molar ratio: MAO/ (RC₅H₄)₂ZrCl₂ | | mol/mol | 100 | 30 | 45 | 24 | 30 | 30 | 266 | 10 | 24 |
| Ratio at which (RC₅H4)₂ ZrCl₂ and 1-decene are blended with each other | | nmol/L | 0.16 | 0.16 | 0.16 | 0.1 | 0.16 | 0.16 | 2.48 | 0.5 | 0.1 |

[Table 7]

**Table 7**

| | | | Example | | | | | | Comparative Example | | |
|---|---|---|---|---|---|---|---|---|---|---|---|
| | | | 25 | 26 | 27 | 28 | 29 | 30 | 4 | 5 | 6 |
| Oligomer yield | | % | 86 | 94 | 87.2 | 92.4 | 79 | 88 | 94 | 90 | 25 |
| Catalytic activity (oligomer amount) | (RC₅H₄)₂ ZrCl₂ (g) basis | kg/g (cat) | 13.6 | 14.8 | 13.8 | 23.3 | 12.5 | 13.9 | 0.96 | 4.54 | 6.3 |
| | MAO (g) basis | kg/g (MAO) | 0.7 | 2.5 | 1.5 | 4.9 | 2.1 | 2.3 | 0.018 | 2.3 | 1.3 |
| Selectivity | Dimer | % | 49 | 42 | 32 | 42 | 28 | 28 | 60 | 77 | 46 |
| | Trimer | % | 17 | 11 | 11 | 24 | 10 | 12 | 23 | 15 | 21 |
| | Tetramer | % | 10 | 7 | 7 | 12 | 7 | 8 | 9 | 5 | 9 |
| | Pentamer | % | 6 | 5 | 5 | 7 | 5 | 6 | 3 | 2 | 5 |
| | Hexamer or more | % | 18 | 35 | 45 | 15 | 50 | 46 | 5 | 1 | 19 |
| Catalyst residue | Cl | ppm by mass | <2 | <2 | <2 | <2 | <2 | <2 | 5 | <2 | <2 |
| | Al | ppm by mass | <2 | <2 | <2 | <2 | <2 | <2 | 15 | <2 | <2 |
| | Zr | ppm by mass | <2 | <2 | <2 | <2 | <2 | <2 | 6 | <2 | <2 |

### [Example 31]

After the monomer and the dimer had been removed from the decalcified liquid obtained in Example 25 under reduced pressure, 1 mass% of a palladium/alumina catalyst (product carrying 5% of Pd) was added to the stainless steel autoclave replaced with nitrogen and having an internal volume of 5 L, and the mixture was subjected to a reaction at 85°C for 5 hours while 0.8 MPa of hydrogen was continuously supplied and the mixture was stirred. After that, the above catalyst was removed by filtration, and the resultant hydrogenated liquid was fractionated with a simple distillation apparatus at 1.33 Pa and 200 to 270°C. A fraction mainly formed of a trimer (formed of 90.1 mass% of the trimer, 9.3 mass% of a tetramer, and 0.5 mass% of a pentamer) out of the resultant fractions was collected. The fraction had an average degree of polymerization of 3.0, 30.1 short-chain branches per 1,000 carbon atoms, and 0.90 short-chain branch on average per molecule of an oligomer. In addition, it was revealed that the kinds of short-chain branching groups included 98.6 mol% of methyl group branches and 1.4 mol% of ethyl group branches, and were hence substantially formed of the methyl group branches.
The fraction had a kinematic viscosity at 40°C of 14.45 mm²/s, a kinematic viscosity at 100°C of 3.55 mm²/s, a viscosity index of 129, a pour point of lower than -50C, a flash point of 240°C, and an amount of evaporation (Noack) of 10.7 mass%.

### [Example 32]

A fraction mainly formed of a tetramer (formed of 10.5 mass% of a trimer, 65.6 mass% of the tetramer, 20.2 mass% of a pentamer, and 3.7 mass% of a hexamer or more) out of the fractions obtained by fractionation after the hydrogenation in Example 31 was collected. Table 8 shows the properties of the fraction.

### [Example 33]

A fraction mainly formed of a pentamer (formed of 0.3 mass% of a trimer, 22.1 mass% of the tetramer, 56.0 mass% of a pentamer, and 21.7 mass% of a hexamer or more) out of the fractions obtained by fractionation after the hydrogenation in Example 31 was collected. Table 8 shows the properties of the fraction.

### [Example 34]

The residue after the hydrogenation and fractionation in Example 31 was examined (the residue was formed of 5.0 mass% of a tetramer, 12.8 mass% af a pentamer, and 82.2 mass% af a hexamer or more). Table 8 shows the properties of the residue.

### [Example 35]

The decalcified liquid obtained in Example 26 was hydrogenated and fractionated in the same manner as in Examples 31, and the residue after the fractionation was examined (the residue was formed of 9.9 mass% of a tetramer, 10.1 mass% of a pentamer, and 80.0 mass% of a hexamer or more). Table 8 shows the properties of the residue.

### [Comparative Examples 7 to 10]

Commercially available poly-α-olefins were examined for their properties. Table 8 shows the results. It can be found that the poly-α-olefins each have a large number of short-chain branches on average per molecule of an oligomer, and each contain large amounts of ethyl group branches and propyl group branches as compared to Examples 31 to 35. In addition, the poly-α-olefins have large amounts of evaporation and low flash points as compared to Examples 31 to 35, though the poly-α-olefins have kinematic viscosities comparable to or slightly higher than those of Examples 31 to 35. This is probably influenced by differences between the examples and the comparative examples in the kinds and amounts of branching groups.

### [Comparative Example 11]

Dimethylsilyl (2-indenyl)(2-methyl-5-t-butyl-6-phenoxy) tit anium dichloride was synthesized with reference to Japanese Patent No. 3,378,436.
Next, 4 L of 1-decene that had been deaerated by nitrogen bubbling and dehydrated was loaded into a stainless steel autoclave replaced with nitrogen and having an internal volume of 5 L. After that, the temperature of the mixture was increased to 50°C, and 50 ml of a solution of a triisobutylaluminum in toluene with its concentration adjusted to 2.0 mol/L were added to the mixture. Next, 50 ml of a suspension of N,N-dimethlyanilinium tetrakispentafluoroborate in toluene with its concentration adjusted to 20 mmol/L was added to the resultant mixture. Finally, 50 mL of a solution of dimethylsilyl (2-indenyl) (2-methyl-5-t-butyl-6-phenoxy)titanium dichloride in toluene with its concentration adjusted to 20 mmol/L were added to the resultant mixture, and the whole was subjected to a reaction at 50°C for 96 hours.
The reaction was stopped with 1 L of 1% dilute hydrochloric acid, and the reaction product was washed with 200 mL of deionized water twice so that a catalyst component might be decomposed and removed. The analysis of the resultant solution by gas chromatography showed that an oligomer yield was 84% and the selectivity of a dimer or more was as follows: the selectivities of a dimer, a trimer, a tetramer, a pentamer, and a hexamer or more were 17%, 24%, 14%, 10%, and 35%, respectively. In addition, the elemental analysis of the solution showed that the solution contained Cl, Al, and Ti at contents of less than 2 ppm by mass each, and was hence substantially free of any catalyst residue.

The decalcifed liquid was hydrogenated and fractionated in the same manner as in Example 31, and a fraction mainly formed of a trimer (formed of 88.5 mass% of the trimer, 9.9 mass% of a tetramer, and 0.8 mass% of a pentamer) was collected. Table 8 shows the properties of the fraction. It was found that the fraction in Comparative Example 13 had the following properties insufficient for use as a base material for a lubricating oil: the fraction had an extremely small number of short-chain branches and a high pour point as compared to Examples 31 to 35.

### [Comparative Example 12]

A fraction mainly formed of a tetramer (formed of 84.6 mass% of the tetramer, 13.2 mass% of a pentamer, and 1.7 mass% of a hexamer or more) out of the fractions obtained by fractionation after the hydrogenation in Comparative Example 11 was collected. Table 3 shows the properties of the fraction. It was found that the fraction had the following properties insufficient for use as a base material for a lubricating oil: the fraction had an extremely small number of short-chain branches and a high pour point as compared to Examples 31 to 35.

### [Comparative Example 13]

The residue after the hydrogenation and fractionation in Comparative Example 11 was examined (the residue was formed of 27.7 mass% of a tetramer, 28.5 mass% of a pentamer, and 43.3 mass% of a hexamer or more). Table 3 shows the properties of the residue. It was found that the residue had the following properties insufficient for use as a base material for a lubricating oil: the residue had an extremely small number of short-chain branches and a high pour point as compared to Examples 31 to 35.

[Table 8]

**Table 8**

| | | | Example | | | | | Comparative Example | | | | | | |
|---|---|---|---|---|---|---|---|---|---|---|---|---|---|---|
| | | | 31 | 32 | 33 | 34 | 35 | 7 | 8 | 9 | 10 | 11 | 12 | 13 |
| Carbon distribution | 20 carbon atoms (dimer) | mass% | 0.1 | 0.0 | 0.0 | 0.0 | 0.0 | 0.6 | 0.0 | 0.0 | 0.0 | 0.7 | 0.1 | 0.2 |
| | 30 carbon atoms (trimer) | mass% | 90.1 | 10.5 | 0.3 | 0.0 | 0.0 | 84.8 | 30.2 | 1.2 | 0.6 | 88.5 | 0.4 | 0.3 |
| | 40 carbon atoms (tetramer) | mass% | 9.3 | 65.6 | 22.1 | 5.0 | 9.9 | 15.0 | 50.5 | 63.3 | 38.7 | 9.9 | 84.6 | 27.7 |
| | 50 carbon atoms (pentamer) | mass% | 0.5 | 20.2 | 56.0 | 12.8 | 10.1 | 0.4 | 17.3 | 31.6 | 34.7 | 0.8 | 13.2 | 26.5 |
| | 60 or more carbon atoms (hexamer or more) | mass% | 0.0 | 3,7 | 21,7 | 82.2 | 0.0 | 0.0 | 1.9 | 3.9 | 26.0 | 1 | 1.7 | 43.3 |
| Average degree of polymerization | | n | 3,0 | 4.0 | 4.8 | 7.5 | 9.2 | 3.2 | 3.9 | 4.3 | 4.8 | 3.0 | 4.0 | 5.0 |
| Number of short-chainbranches | | short-chain branches/1,0 00 carbon atoms | 30.1 | 22.8 | 18.3 | 12.0 | 9.6 | 42.5 | 36.7 | 35.2 | 39.3 | 4.8 | 3.6 | 2.7 |
| Average number of short-chainbranches per molecule Of oligomer | | Short chain branches | 0.90 | 0.91 | 0.88 | 0.90 | 0.88 | 1.38 | 1.42 | 1.56 | 1.89 | 0.14 | 0.14 | 0.14 |
| Branching group ratio | Methyl group | mol% | 98.6 | 96.9 | 95.8 | 93 | 92 | 59.7 | 61.5 | 64.5 | 66.1 | 91.6 | 83.6 | 73.3 |
| | Ethyl group | mol% | 1.4 | 1.5 | 1.9 | 2.5 | 2.8 | 17.5 | 18.3 | 15.4 | 13.6 | 8.4 | 6.6 | 10.6 |
| | propyl group | mol% | 0 | 1.6 | 2,3 | 4.5 | 5.2 | 22.8 | 20.2 | 20.1 | 20.3 | 0 | 9.8 | 16.1 |
| Kinematic viscosity at 40°C | | mm²/s | 14.45 | 27.87 | 41.78 | 119.1 | 208.1 | 17.31 | 28.8 | 46 | 63 | 14.18 | 28.05 | 49.46 |
| Kinematic viscosity at 100°C | | mm²/s | 3.55 | 5.53 | 7.46 | 16.94 | 27.3 | 3.94 | 5.6 | 7.78 | 9.8 | 3.58 | 5.709 | 8.728 |
| Viscosity index | | - | 129 | 140 | 146 | 155 | 168 | 125 | 136 | 138 | 139 | 139 | 150 | 156 |
| Pour point | | °C | ← 50 | ← 50 | ← 50 | ← 50 | -50 | ← 50 | ← 50 | ← 50 | ← 50 | -37 | -42 | -36 |
| Flash point (COC) | | °C | 240 | 254 | 262 | 278 | 276 | 236 | 240 | 256 | 270 | 246 | - | - |
| amount of evaporation | | mass% | 10.7 | 4.7 | 3.3 | 1.8 | 1.7 | 14.9 | 6.7 | - | 1.6 | - | - | - |

### Industrial Applicability

According to the present invention, there is provided a method of producing an α-olefin oligomer composition by which the amount of a catalyst can be reduced and a timer, a tetramer, and a pentamer can each be produced with a high selectivity. The α-olefin oligomer obtained in the present invention is preferably used as a component for a lubricating oil.

## Claims

1. A method of producing an α-olefin oligomer composition, the method comprising the step of causing molecules of an α-olefin to react with each other in the presence of hydrogen with a catalyst formed by using the following components (A) and (B):
(A) a transition metal compound represented by a general formula (I) :
{C₅R¹R²R³R⁴(A¹R^{a}R^{b}R^{c}) } {C₅R⁵R⁶R⁷R⁸(A²R^{d}R^{e}R^{f}) }MXY (I)
where R¹ to R⁸, (A¹R^{a}R^{b}R^{c}), and (A²R^{d}R^{e}R^{f}) each represent a substituent bonded to a cyclopentadienyl group, R¹ to R⁸ each independently represent a hydrogen atom or a hydrocarbon group having 1, to 10 carbon atoms, R^{a} to R^{f} each independently represent a hydrocarbon group having 1 to 10 carbon atoms, and two or more groups selected from R^{a}, R^{b}, and R^{c} may be bonded to each other to form a ring, or two or more groups selected from R^{d}, R^{e}, and R^{f} may be bonded to each other to form a ring, A¹ and A² each independently represent an element belonging to Group 14 of the periodic table, M represents a transition element belonging to Group 4 of the periodic table, and X and Y each represent a covalent ligand or an ionic bond ligand; and
(B) a co-catalyst containing aluminum.

2. A method of producing an α-olefin oligomer composition, the method comprising the step of causing molecules of an α-olefin to react with each other with a catalyst formed by using the following components (A) and (B) in an aluminum amount in a range of 1.89x10⁻⁵ to 5.67x10⁻² mol per mol of the α-olefin at a hydrogen pressure in a range of 1 to 50 kPa (G):
(A) a transition metal compound represented by a general formula (I):
{C₅R¹R²R³R⁴ (A¹R^{a}R^{b}R^{c}) } {C₅R⁵R⁶R⁷R⁸ (A²R^{d}R^{e}R^{f}) }MXY (I)
where R¹ to R⁸, (A¹R^{a}R^{b}R^{c}), and (A²R^{d}R^{e}R^{f}) each represent a substituent bonded to a cyclopentadienyl group, R¹ to R⁸ each independently represent a hydrogen atom or a hydrocarbon group having 1 to 10 carbon atoms, R^{a} to R^{f} each independently represent a hydrocarbon group having 1 to 10 carbon atoms, and two or more groups selected from R^{a}, R^{b}, and R^{c} may be bonded to each other to form a ring, or two or more groups selected from R^{d}, R^{e}, and R^{f} may be bonded to each other to form a ring, A¹ and A² each independently represent an element belonging to Group 14 of the periodic table, M represents a transition element belonging to Group 4 of the periodic table, and X and Y each represent a covalent ligand or an ionic bond ligand; and
(B) a co-catalyst containing aluminum.

3. The method of producing an α-olefin oligomer composition according to claim 2, wherein the co-catalyst containing aluminum as the component (B) comprises an organic aluminum oxy compound and/or an organic aluminum compound.

4. The method of producing an α-olefin oligomer composition according to claim 2, wherein the transition metal compound as the component (A) comprises a transition metal compound represented by a general formula (II):
{C₅H₄(A³R^{g}R^{h}Rⁱ) }₂ZrCl₂ (II)
wherein R^{g}, R^{h}, and Rⁱ each independently represent a hydrocarbon group having 1 to 10 carbon atoms, and two or more of the groups may be bonded to each other to form a ring, and A³ represents an element belonging to Group 14 of the periodic table.

5. The method of producing an α-olefin oligomer composition according to claim 2, wherein the transition metal compound as the component (A) comprises a transition metal compound represented by a general formula (III):
{C₅H₄ (CR^{j}R^{k}R¹) l}₂ZrCl₂ (III)
wherein R^{j}, R^{k}, and R¹ each independently represent a hydrocarbon group having 1 to 10 carbon atoms, and two or more of the groups may be bonded to each other to form a ring.

6. The method of producing an α-olefin oligomer composition according to claim 2, wherein the transition metal compound as the component (A) comprises a transition metal compound represented by a formula (IV):
{C₅H₄ (CMe₃) }₂ZrCl₂ (IV).

7. The method of producing an α-olefin oligomer composition according to claim 2, wherein the α-olefin comprises 1-decene.

8. The method of producing an α-olefin oligomer composition according to claim 2, wherein the α-olefin oligomer composition comprises an α-olefin oligomer composition satisfying the following relationships: A>B and A>C when a ratio of a trimer to a dimer on a mass basis is represented by A, a ratio of a tetramer to the trimer on a mass basis is represented by B, and a ratio of a pentamer to the tetramer on a mass basis is represented by C.

9. An α-olefin oligomer composition obtained by the method according to claim 2.

10. A method of producing a hydrogenated α-olefin oligomer composition, the method comprising the step of subj ecting an α-olefin oligomer composition obtained by the method according to claim 2 to a hydrogenation treatment.

11. A hydrogenated α-olefin oligomer composition obtained by the method according to claim 10.

12. A lubricating oil composition comprising the α-olefin oligomer composition according to claim 9 and/or the hydrogenated α-olefin oligomer composition according to claim 11.

13. A method of producing an α-olefin oligomer, the method comprising the step of polymerizing molecules of an α-olefin having 3 to 14 carbon atoms in the presence of hydrogen by using a metallocene compound represented by a general formula (VII) and a methylaluminoxane at a molar ratio (methylaluminoxane/metallocene compound) of 15 to 110:
(RC₅H₄)₂MX₂ (VII)
wherein R represents a hydrogen atom or a hydrocarbon group having 1 to 10 carbon atoms, M represents a transitionmetal element belonging to Group 4 of the periodic table, and X represents a covalent ligand or an ionic bond ligand.

14. The production method according to claim 13, wherein a ratio at which the metallocene compound represented by the general formula (VII) and the α-olefin are blended with each other [metallocene compound (mmol)/α-olefin (L)] falls within a range of 0.01 to 0.4.

15. The production method according to claim 13, wherein M in the general formula (VII) represents zirconium.

16. The production method according to claim 13, wherein a hydrogen pressure falls within a range of 0.1 to 50 kPa.

17. The production method according to claim 13, wherein the α-olefin having 3 to 14 carbon atoms comprises an α-olefin selected from 1-octene, 1-decene, and 1-dodecene.

18. The production method according to claim 13, wherein the α-olefin having 3 to 14 carbon atoms comprises 1-decene.

19. The production method according to claim 13, wherein a selectivity of a trimer or more is 50% or more.

20. An α-olefin oligomer obtained by the production method according to claim 13.

21. A method of producing a purified hydrogenated α-olefin oligomer, the method comprising the steps of:
producing an α-olefin oligomer by the production method according to claim 13;
hydrogenating the α-olefin oligomer to produce a hydrogenated α-olefin oligomer; and
distilling the hydrogenated α-olefin oligomer to obtain a fraction having a kinematic viscosity at 100°C of 3 to 35 mm²/s.

22. A purified hydrogenated α-olefin oligomer obtained by the production method according to claim 21.
